# EUROPEAN PATENT SPECIFICATION

(11) **EP 0 728 218 B1**
(45) Date of publication and mention of the grant of the patent: **02.01.2008**
(21) Application number: 95904104.7
(22) Date of filing: 14.11.1994
(51) Int. Cl.: C12Q 1/68, C07H 21/00

(54) **HYBRIDIZATION PROBES FOR NUCLEIC ACID DETECTION, UNIVERSAL STEMS, METHODS AND KITS**
HYBRIDISIERUNGSPROBE ZUR NUKLEINSÄUREDETEKTION, UNIVERSELLE STAMMSTRUKTUREN, METHODEN UND REAGENTIENSÄTZE
SONDES D'HYBRIDATION POUR LA DETECTION D'ACIDES NUCLEIQUES, SOUCHES UNIVERSELLES, METHODES ET MATERIELS

(30) Priority: 12.11.1993 US 152006
(43) Date of publication of application: 28.08.1996
(60) Divisional application: 07075511.1
(73) Proprietor: PHRI Properties, Inc., Newark, NJ 07107-3001 (US)
(72) Inventor: TYAGI, Sanjay, New York, NY 10019 (US); KRAMER, Fred R., Riverdale, NY 10463 (US); LIZARDI, Paul M., Wallingford, CT 06492 (US)
(74) Representative: Bizley, Richard Edward
(86) International application number: PCT/US1994/013415
(87) International publication number: WO 1995/013399

(56) References cited:
- EP-A- 0 229 943
- EP-A- 0 601 889
- WO-A-92/14845
- US-A- 4 766 062
- US-A- 5 118 801
- CARDULLO R A ET AL: "DETECTION OF NUCLEIC ACID HYBRIDIZATION BY NONRADIATIVE FLUORESCENCE RESONANCE ENERGY TRANSFER" PROCEEDINGS OF THE NATIONAL ACADEMY OF SCIENCES OF USA, vol. 85, no. 23, 1 December 1988, pages 8790-8794, XP000453537
- BIOCHEMISTRY, Volume 29, issued 1990, J.P. COOPER et al., "Analysis of Fluorescence Energy Transfer in Duplex and Branched DNA Molecules", pages 9261-9268
- TETRAHEDRON LETTERS, Volume 31, No. 45, issued 1990, G.T. WANG et al., "Design and Synthesis of New Fluorogenic HIV Protease Substrates Based on Resonance Energy Transfer", pages 6493-6496.

## Description

The present invention relates to the field of assays which involve nucleic acid hybridization probes. Assays are useful for the detection of specific genes, gene segments, RNA molecules and other nucleic acids. Such assays are used clinically, for e.g., tissue, blood and urine samples, as well as in food technology, agriculture and biological research.

### Background of the Invention

Nucleic acid hybridization probes are used to detect specific target sequences in a complex mixture. Conventional, heterogeneous, hybridization assays, such as those described in Gillespie and Spiegelman (1965), typically comprise the following steps: immobilization of at least the target nucleic acid on paper, beads, or plastic surfaces, with or without using capture probes; addition of an excess of labelled probes that are complementary to the sequence of the target; hybridization; removal of unhybridized probes; and detection of the probes remaining bound to the immobilized target.

EP 0 229 943 discloses probes labeled with a donor fluorophore and an acceptor fluorophore, wherein the fluorophores are separated by a critical distance to maximize fluorescence resonance energy transfer such that the probes can be excited at a short wavelength but detected at a long wavelength.

Unhybridized probes are removed by extensive washing of the hybrids. This is generally the most time-consuming part of the procedure, and often utilizes complex formats such as sandwich hybridization. The use of solid surfaces lengthens the time it takes for hybridization by restricting the mobility of, or access to, the target. The large area presented by the solid surfaces nonspecifically retains unhybridized probes, leading to background signal. Additionally, solid surfaces may interfere with signal from the probes. The requirement that the probe-target hybrids be isolated precludes in vivo detection and concurrent detection of nucleic acids during synthesis reactions (real-time detection).

Several solution-phase detection schemes, sometimes referred to as homogeneous assays, are known. By "homogeneous" we mean assays that are performed without separating unhybridized probes from probe-target hybrids. These schemes often utilize the fact that the fluorescence of many fluorescent labels can be affected by the immediate chemical environment. One such scheme is described by Heller et al. (1983) and also by Cardullo et al. (1988). It uses a pair of oligodeoxynucleotide probes complementary to contiguous regions of a target DNA strand. One probe contains a fluorescent label on its 5' end and the other probe contains a different fluorescent label on its 3' end. When the probes are hybridized to the target sequence, the two labels are very close to each other. When the sample is stimulated by light of an appropriate frequency, fluorescence resonance energy transfer ("FRET") from one label to the other occurs. This energy transfer produces a measurable change in spectral response, indirectly signaling the presence of target. The labels are sometimes referred to as FRET pairs. However, the altered spectral properties are subtle, and the changes are small relative to background signal. Monitoring requires sophisticated instruments, and, even so, sensitivity is limited. Moreover, the hybridization signal is, in some cases, a negative one; i.e., the presence of target results in a reduction in the amount of fluorescence measured at a particular wavelength.

This technique requires that two unassociated probes bind simultaneously to a single-stranded target sequence. The kinetics of this tri-molecular hybridization are too slow for this technique to be suitable for real-time detection. The requirement that target be single-stranded makes the technique unsuitable for in vivo detection of double-stranded nucleic acids.

Another solution-phase scheme also utilizes a pair of oligodeoxynucleotide probes. However, here the two probes are completely complementary both to each other and to complementary strands of a target DNA (Morrison, 1987; Morrison, 1989; Morrison et al., 1989; Morrison and Stols, 1993). Each probe includes a fluorophore conjugated to its 3' end and a quenching moiety conjugated to its 5' end.

When the two oligonucleotide probes are annealed to each other, the fluorophore of each probe is held in close proximity to the quenching moiety of the other probe. If the fluorescent label is then stimulated by an appropriate frequency of light, the fluorescence is quenched by the quenching moiety. However, when either probe is bound to a target, the quenching effect of the complementary probe is absent. The probes are sufficiently long that they do not self-quench when target-bound.

In this type of assay, there are two opposing design considerations. It is desirable to have a high concentration of probes to assure that hybridization of probes to target is rapid. It is also desirable to have a low concentration of probes so that the signal from probes bound to target is not overwhelmed by background signal from probes not hybridized either to target or other probes. This situation necessitates waiting a relatively long time for the background fluorescence to subside before reading the fluorescent signal.

An assay according to this scheme begins by melting a mixture of probes and sample that may contain target sequences. The temperature is then lowered, leading to competitive hybridization. Some probes will hybridize to target, if present; some will hybridize to complementary probes; and some will not hybridize and create background signal. A parallel control assay is run with no target, giving only a background level of fluorescence. If the sample contains sufficient target, a detectably higher level of residual fluorescence is obtained.

With this scheme it is necessary to delay reading the residual fluorescence for a considerable time to permit nearly all the excess probes to anneal to their complements. Also, a parallel control reaction must be performed. Additionally, a low concentration of probes is used to reduce the fluorescent background. Thus, kinetics are poor and result in an inherently slow assay. That precludes real-time detection. These problems are particularly severe for double-stranded targets. The probes, as well as the targets, need to be melted, rendering the assay unsuitable for use in vivo.Also, the signal is not only residual, it is a differential signal from comparison to an external control.

EP 0 601 889 discloses that the target-recognition and competitive sequences of a probe pair can be combined into a strand containing a nucleotide spacer separating those sequences. In these probes each sequence is labeled, one with donor fluorophore and the other with an acceptor fluorophore. Absent target the two sequences hybridize, and there is energy transfer. In use the probe is contacted with target at a temperature sufficiently high to disrupt the intra-probe hybrid such that there are single-stranded species of both target and probe and a lack of secondary structures such as hairpins. The target then competes with the probe's quenching strand or strands, herein referred to as a competitive arm or arms, to hybridize to the probe's target recognition sequence.

Another solution-phase scheme utilizing the phenomenon known as strand displacement is described by Diamond et al., 1988. Typically, these assays involve a bimolecular nucleic acid probe complex. A shorter single-strand comprising a subset of the target sequence is annealed to a longer probe single-strand which comprises the entire target binding region of the probe. The probe complex reported thus comprises both single-stranded and double-stranded portions. The reference proposed that these probe complexes may further comprise either a "P label attached to the shorter strand or a fluorophore and a quencher moiety which could be held in proximity to each other when the probe complex is formed.

It is stated that in assays utilizing these probe complexes, target detection is accomplished by a two-step process. First, the single-stranded portion of the complex hybridizes with the target. It is described that target recognition follows thereafter when, through the mechanism of branch migration, the target nucleic acid displaces the shorter label-bearing strand from the probe complex. The label-bearing strand is said to be released into solution, from which it may be isolated,and detected. In an alternative arrangement reported as a ³²P labeled probe for a capture procedure, the two single-stranded nucleic acids are linked together into a single molecule.

These strand-displacement probe complexes have drawbacks. The mechanism is two-step, in that the probe complex must first bind to the target and then strand-displacement, via branch migration, must occur before a target is recognized and a signal is generated. Bimolecular probe complexes are not reported to form with high efficiency, resulting in probe preparations wherein the majority of the target binding regions may not be annealed to a labeled strand. This may lead to competition between label-bearing and label-free target binding regions for the same target sequence. Additionally, there may be problems with non-specific fall-off of labeled strands. Moreover, the displaced labeled strand may need to be separated from the unhybridized probe complexes before a signal may be detected. This requirement would make such a probe complex unsuitable for a homogeneous assay.

Published Japanese Patent Application No. 3-285221 discloses having either a two-part target complement sequence separated by an intervening sequence or a one-part target complement sequence flanked by stem-forming sequences. In both cases the portion(s) other than the target complement sequence carry a donor fluorophore and an acceptor fluorophore. In use the probes are mixed with a target, after which the mixture is heated to at least 60°C and then slowly cooled over at least five minutes, after which a change in fluorescence is detected.

It is an object of the present invention to overcome the limitations, discussed above, of conventional hybridization probes and assays and of existing homogeneous hybridization probes and assays.

Another object of this invention is the use of hybridization probes that generate a signal upon hybridization with a target, nucleic acid sequence but exhibit little or no signal generation when unhybridized, and assays using these probes.

Further objects of this invention are kits that can be used to make such nucleic acid probes specific for target sequences of choice.

A further object of this invention is homogeneous assays using such probes.

A further object of this invention is hybridization probes and rapid methods wherein detection is performed quickly and without delay.

A further object of this invention is hybridization probes and methods that can detect nucleic acids in vivo.

A further object of this invention is hybridization probes and methods that can detect nucleic acids in situ.

A further object of this invention is hybridization probes and methods that can detect nucleic acid target sequences in nucleic acid amplification and other synthesis reactions in real-time mode.

A further object of this invention is hybridization probes and assays that permit detection of nucleic acid targets without the use of expensive equipment.

In order to realize the full potential of the process of hybridization in the field of diagnostics and research, a technique is needed for monitoring hybridization in solutions with probes having little or no signal of their own yet producing a detectable signal when hybridized to a target. Preferably, the probe should permit monitoring of the progress of reactions that produce nucleic acids with either linear or exponential kinetics. Also, the probe should allow detection of nucleic acids in vivo (and in situ) without the destruction of the tissue or cell. Of course, the probe should also be useful in conventional hybridization assays. Additionally, the assays should permit very sensitive detection of targets either directly or in conjunction with amplification techniques. Also preferably, the probe should be capable of generating a hybridization signal detectable by the naked eye. Finally, the probes should permit detection of different targets in a single assay. Objects of this invention are nucleic acid hybridization assays and probes that satisfy all or nearly all of these requirements.

Some embodiments of subject-matter relating to the process of the invention as claimed are :-
1. A labeled unitary probe for detecting at least one nucleic acid strand containing a preselected nucleic acid target sequence under preselected assay conditions including a detection temperature, said probe comprising:
   a single-stranded target complement sequence having from 10 to about 140 nucleotides, having a 5' terminus and a 3' terminus, and being complementary to the target sequence;
   a stem duplex 3 to 25 nucleotides in length consisting of a 5' arm sequence adjacent to and covalently linked to said 5' terminus and a 3' arm sequence adjacent to and covalently linked to said 3' terminus, said duplex having a melting temperature above said detection temperature under the preselected assay conditions; and
   at least one label pair, each pair comprising a first label moiety conjugated to the probe in the vicinity of said 5' arm sequence and a second label moiety proximate to said first label moiety and conjugated to the probe in the vicinity of said 3' arm sequence,
   said probe having, under said preselected assay conditions in the absence of said target sequence, a characteristic signal whose level is a function of the degree of interaction of said first and second label moieties, said signal having a first level at 10°C below said melting temperature, a second level at 10°C above said melting temperature and a third level at said detection temperature,
   wherein under the preselected assay conditions at the detection temperature and in the presence of an excess of said target sequence, hybridization of the target complement sequence to the target sequence alters the level of said characteristic signal from said third level toward the second level by an amount of at least ten percent of the difference between the first and second levels.
2. A probe according to embodiment 1 wherein said unitary probe is a unimolecular probe.
3. A probe according to embodiment 1 wherein said unitary probe is a bimolecular probe consisting of a first molecule containing approximately half of said target complement sequence including said 5' terminus, the 5' arm sequence and the first label moiety; and a second molecule containing approximately half of said target complement sequence including said 3*'* terminus, the 3' arm sequence and the second label moiety.
4. A probe according to embodiment 1 wherein said at least one label pair is a FRET pair.
5. A probe according to embodiment 1 wherein said melting temperature is at least 5°C above said detection temperature.
6. A probe according to embodiment 5 wherein said at least one label pair is a FRET pair.
7. A unimolecular probe according to embodiment 6.
8. A probe according to embodiment 6 wherein said 5' and 3' arm sequences are immediately adjacent to said target complement sequence.
9. A unimolecular probe according to embodiment 8.
10. A probe according to embodiment 6 wherein said first and second label moieties are covalently linked into said unitary probe.
11. A unimolecular probe according to embodiment 10.
12. A probe according to embodiment 6 wherein said melting temperature is at least 10°C above said detection temperature.
13. A unimolecular probe according to embodiment 12.
14. A probe according to embodiment 6 wherein said FRET pair is a fluorescer and a quencher.
15. A unimolecular probe according to embodiment 14.
16. A probe according to embodiment 7 wherein said FRET pair is EDANS and DABCYL.
17. A probe according to embodiment 1 wherein said 5' and 3' arm sequences are covalently linked directly to said target complement sequence.
18. A unimolecular probe according to embodiment 17.
19. A probe according to embodiment 1 wherein at least one of said 5' and 3' arm sequences is at least partially complementary to the nucleic acid strand containing the target sequence.
20. A unimolecular probe according to embodiment 19.
21. A probe according to embodiment 1 wherein each of said 5' and 3' arm sequences comprises neighboring complementary sequences of at least three nucleotides.
22. A unimolecular probe according to embodiment 21.
23. A probe according to embodiment 1 wherein the probe sequences are selected from the group consisting of DNA, RNA, and mixtures of DNA and RNA.
24. A probe according to embodiment 1 wherein said first and second label moieties are covalently linked into said unitary probe.
25. A probe according to embodiment 24 wherein said first and second label moieties are covalently linked through an alkyl spacer.
26. A unimolecular probe according to embodiment 25.
27. A probe according to embodiment 1 tethered to a solid surface.
28. A unimolecular probe according to embodiment 27.
29. A probe according to embodiment 1 wherein said melting temperature is at least 5°C above said detection temperature, wherein said 5' and 3' arm sequences are covalently linked directly to said target complement sequence, wherein said first and second label moieties are covalently linked to said 5' and 3' arm sequences, and said at least one label pair is a fluorescer and a quencher.
30. A unimolecular probe according to embodiment 29.
31. A probe according to embodiment 29 tethered to a solid support.
32. A unimolecular probe according to embodiment 31.
33. A probe according to embodiment 29 wherein said target complement sequence has from 20 to 60 nucleotides.
34. A unimolecular probe according to embodiment 33.
35. A probe according to embodiment 34 tethered to a solid support.
36. A unitary hybridization probe for the detection of at least one nucleic acid target comprising a preselected target sequence in a preselected nucleic acid hybridization assay, said probe being capable of assuming a closed conformation and an open conformation, comprising: '
   a) a-target complement sequence of from 10 to about 140 nucleotides complementary to said preselected nucleic acid target sequence, having a 5' terminus and a 3' terminus,
   b) an affinity pair comprising a first affinity moiety covalently linked to said 5' terminus and a second affinity moiety covalently linked to said 3' terminus, said affinity pair interacting sufficiently to hold said probe in the closed conformation in said preselected assay in the absence of said nucleic acid target, and
   c) a label pair comprising a first label moiety conjugated to said probe in the vicinity of said first affinity moiety and a second label moiety conjugated to said probe in the vicinity of said second affinity moiety, said label moieties interacting to affect a measurable characteristic of at least one of them when said probe is in the closed conformation,
   wherein hybridization of said target complementary sequence to said target sequence in said preselected assay causes said probe to assume its open conformation, in which said label moieties do not so interact.
37. A probe according to embodiment 36 wherein said unitary probe is a unimolecular probe.
38. A probe according to embodiment 36 wherein said unitary probe is a bimolecular probe consisting of a first molecule containing approximately half of said target complement sequence including said 5' terminus, the first affinity moiety and the first label moiety; and a second molecule containing approximately half of said target complement sequence including said 3' terminus, the second affinity moiety and the second label moiety.
39. A probe according to embodiment 36 wherein said first label moiety is conjugated to said first affinity moiety and said second label moiety is conjugated to said second affinity moiety.
40. A unimolecular probe according to embodiment 39.
41. A probe according to embodiment 36 wherein said affinity pair comprises complementary oligonucleotide arm sequences 3 to 25 nucleotides in length.
42. A probe according to embodiment 41 wherein said first label moiety is covalently linked to said first affinity moiety and said second label moiety is covalently linked to said second affinity moiety.
43. A unimolecular probe according to embodiment 42.
44. A probe according to embodiment 39 wherein said label pair comprises a FRET pair.
45. A unimolecular probe according to embodiment 44.
46. A probe according to embodiment 36 wherein said affinity pair comprises an antibody and an antigen.
47. A probe according to embodiment 36 tethered to a solid surface.
48. A unimolecular probe according to embodiment 47.
49. A universal stem comprising:
   a first oligonucleotide of 5 to 20 nucleotides having a first covalently reactive group at its 3' terminus and having conjugated to it at a location other than its 3' terminus at least one first label moiety of a first label pair, and
   a second oligonucleotide, complementary to said first oligonucleotide, of 5 to 20 nucleotides having a second covalently reactive group at its 5' terminus and having conjugated to it at a location other than its 5' terminus at least one second label moiety of said first label pair, said label moieties being proximate when said oligonucleotides are hybridized to one another,
   wherein, at least one label moiety of said first label pair affects a physically measurable characteristic of the other when said first and second label moieties are proximate to one another to an extent detectably different from when they are not proximate to one another.
50. A universal stem according to embodiment 49 wherein said first and second oligonucleotides are selected from the group consisting of DNA, RNA and mixtures of DNA and RNA.
51. A universal stem according to embodiment 50 wherein said first covalently reactive group and said second covalently reactive group are each selected from the group consisting of hydroxyl, phosphate, sulfhydryl, amino, alkyl phosphate, alkyl amino and hydroxy alkyl.
52. A universal stem according to embodiment 49 wherein each of said first and second oligonucleotides includes neighboring complementary sequences of at least three nucleotides.
53. A universal stem according to embodiment 49 wherein said first label pair is a FRET pair.
54. A universal stem according to embodiment 49 wherein said first and second oligonucleotides hybridize to one another providing a ligatable.overhang in which one of said 5' terminus and said 3' terminus extends beyond the other by at least two nucleotides.
55. A universal stem according to embodiment 54 wherein said first label pair is a FRET pair.
56. A universal stem according to embodiment 49 wherein said first and second oligonucleotides are separately packaged in a pair of containers.
57. A universal stem according to embodiment 49 wherein one of said first and second oligonucleotides is tethered to a solid surface.
58. A kit for using a universal stem to construct at least one probe according to embodiment 1 comprising:
   a universal stem according to embodiment 49, and
   information sufficient for covalently linking to said stem a target complement sequence according to embodiment 1.
59. A kit according to embodiment 58 wherein the first label pair of said universal stem is a FRET pair.
60. A kit according to embodiment 58 wherein the first and second oligonucleotides of said universal stem are separately packaged.
61. A kit according to embodiment 60 wherein said information is sufficient for covalently linking to the first oligonucleotide a first nucleic acid strand containing approximately half of a target complement sequence according to embodiment 1 including its 3' terminus and for covalently linking to the second oligonucleotide a second nucleic acid strand containing approximately half of said target complement sequence including its 5' terminus.
62. A kit according to embodiment 61 wherein one of said first and second oligonucleotides is tethered to a solid surface.
63. A kit according to embodiment 58 wherein the first and second oligonucleotides of said stem hybridize to one another providing a ligatable overhang in which one of said 5' terminus and said 3' terminus extends beyond the other by at least two nucleotides, and wherein said information is sufficient for ligating to said stem a self-hybridizing single strand that is complementary to said overhang and includes a target complement sequence according to embodiment 1.
64. A kit according to embodiment 63 containing at least two stems having different melting temperatures under the preselected assay conditions.
65. A kit according to embodiment 64, wherein said stems have a common first or second oligonucleotide.
66. A kit according to embodiment 63 wherein the first label pair of said universal stem is a FRET pair.
67. A kit according to embodiment 63 wherein each of said first and second oligonucleotides of said stem includes neighboring complementary sequences of at least three nucleotides.
68. A kit according to embodiment 63 wherein one of said first and second oligonucleotides is tethered to a solid surface.
69. An assay for detecting at least one nucleic acid strand containing a preselected target sequence under preselected assay conditions including a detection temperature, comprising the steps of:
   adding to a sample a unitary probe according to embodiment 1 under said preselected assay conditions, and
   ascertaining any change in the level of said characteristic signal at said detection temperature.
70. An assay according to embodiment 69 wherein said step of ascertaining comprises measuring.
71. A real-time assay according to embodiment 70.
72. A real-time assay according to embodiment 69.
73. An assay according to embodiment 70, wherein said step of ascertaining comprises measuring as a function of time.
74. An assay according to embodiment 70 further comprising a step of adding to a target-less control the probe according to embodiment 1 under said preselected assay conditions and measuring any change in said level for said control, wherein said step of ascertaining includes calculating a difference between the change in level for said control and the change in level for said sample.
75. An assay according to embodiment 69 further comprising a step of adding to a target-less control the probe according to embodiment 1.
76. An assay according to embodiment 69 wherein said probe is a bimolecular probe according to embodiment 3, and the assay is performed entirely below the melting temperature of said probe.
77. An assay according to embodiment 69 wherein the melting temperature of said probe is at least 5°C above said detection temperature, wherein said 5' and 3' arm sequences are covalently linked directly to said target complement sequence, wherein said first and second label moieties are covalently linked to said 5' and 3' arm sequences, and said at least one label pair is a fluorescer and a quencher.
78. A real-time assay according to embodiment 77.
79. An assay according to embodiment 77 wherein said probe is a bimolecular probe according to embodiment 3, and the assay is performed entirely below the melting temperature of said probe.
80. An assay according to embodiment 69 wherein the step of ascertaining is quantitative.
81. An assay according to embodiment 69 wherein said probe is a unimolecular probe.
82. An assay according to embodiment 81 wherein the step of ascertaining is quantitative.
83. An assay according to embodiment 81 further comprising a step of adding to a target-less control said unimolecular probe according to embodiment 2.
84. An assay according to embodiment 81 wherein the melting temperature of said probe is at least 5°C above the detection temperature, wherein said 5' and 3' arm sequences are covalently linked directly to said target complement sequence, wherein said first and second label moieties are covalently linked to said 5' and 3' arm sequences, and said at least one label pair is a fluorescer and a quencher.
85. An assay according to embodiment 81 wherein said nucleic acid target sequence is a product of an amplification reaction, and wherein said step of adding said probe to a sample precedes completion of said amplification reaction.
86. An assay according to embodiment 85 further comprising a step of adding said probe to a negative control, which step precedes completion of said amplification reaction on said negative control.
87. A real-time assay according to embodiment 86.
88. An assay according to embodiment 87 wherein said step of ascertaining includes calculating a difference between the change in level for said control and the change in level for said sample.
89. An assay according to embodiment 85 wherein said step of ascertaining includes measuring repeatedly.over time during said amplification reaction.
90. An assay according to embodiment 89 wherein the melting temperature of said probe is at least 5°C above the detection temperature, wherein said 5' and 3' arm sequences are covalently linked directly to said target complement sequence, wherein said first and second label moieties are covalently linked to said 5' and 3' arm sequences, and said at least one label pair is a fluorescer and a quencher.
91. An assay according to embodiment 85 wherein said amplification reaction is amplification of an RNA reporter by an RNA polymerase.
92. An assay according to embodiment 85 wherein said amplification reaction is a PCR reaction.
93. An assay according to embodiment 85 wherein said amplification reaction is an SDA reaction.
94. An assay according to embodiment 85 wherein said label pair is a FRET pair.
95. An assay according to embodiment 69 wherein said probe according to embodiment 1 is tethered to a solid surface.
96. An assay according to embodiment 95 including at least one additional tethered probe according to this invention having a different target complement sequence and linked to the same support surface at a predetermined location.
97. An assay for detecting at least one nucleic acid target containing a preselected target sequence comprising the steps of:
   adding to a sample a unitary probe according to embodiment 36 under said preselected assay conditions, and
   ascertaining any change in the level of said measurable characteristic.
98. An assay according to embodiment 97 wherein said step of ascertaining comprises measuring.
99. An assay according to embodiment 98 wherein said measuring comprises visually comparing to a standard.
100. An assay according to embodiment 97 wherein said nucleic acid target is a product of an amplification reaction, and wherein said probe is unimolecular.
101. An assay according to embodiment 100 wherein said step of adding said probe precedes completion of said amplification reaction.
102. A kit for performing an assay according to embodiment 69 comprising a probe according to embodiment 1 and instructions for performing the assay.
103. A kit according to embodiment 102 further including one or more reagents selected from the group consisting of salts, buffers, nuclease inhibitors, restriction enzymes and denaturants.
104. An amplification kit according to embodiment 102 including one or more components selected from the group consisting of primers, nucleotides, polymerases and polymerase templates.
105. An amplification kit according to embodiment 102 wherein the probe is unimolecular.
106. A vital stain assay kit according to embodiment 102 including one or more components selected from the group consisting of permeabilizing agents, liposome precursors and counterstains.
107. An in situ assay kit according to embodiment 102 including one or more-components selected from the group consisting of fixatives, dehydrating agents, proteases, counterstains and detergents.
108. A field kit according to embodiment 102 wherein said probe is a tethered probe.
109. A kit according to embodiment 108 including at least one additional tethered probe according to this invention having a different target complement sequence and linked to the same support surface at a predetermined location.
110. A kit according to embodiment 108 including a positive control probe according to this invention.
111. A kit according to embodiment 108 wherein said probe is unimolecular.

Probes for use in accordance with this invention are "unitary", by which we mean either bimolecular probes linked as a pair and operable in an assay as linked, or unimolecular, single strands. They comprise at least: a single-stranded nucleic acid sequence that is complementary to a desired target nucleic acid, herein referred to as a "target complement sequence;" 5' and 3' regions flanking the target complement sequence that reversibly interact by means of complementary nucleic acid sequences; and interactive label moieties for generating a signal. Preferred probes for use in this invention include complementary nucleic acid sequences, or "arms," that reversibly interact by hybridizing to one another under the conditions of detection when the target complement sequence is not bound to the target. Where the unitary probe is unimolecular, all the above components are in one molecule. When the unitary probe is bimolecular, half, or roughly half, of the target complement sequence, one member of the affinity pair and one member of the label pair are in each molecule.

The signal generating label moieties are "pairs" matched such that at least one label moiety can alter at least one physically measurable characteristic of another label moiety when in close proximity but not when sufficiently separated. The label moieties are conjugated to the probe such that the proximity of the label moieties to each other is regulated by the status of the interaction of the affinity pair. In the absence of target, the label moieties are held in close proximity to each other by the linking interaction of the affinity pair. We refer to this conformation as the "closed" state. When the target-indicating detectable signal is not generated in the closed state, which is the fact with most embodiments, we say that the closed state is the "off" state.

When the target complement sequence hybridizes to its target, a conformational change occurs in the unitary probe, separating the affinity pair and, consequently, the label moieties. We refer to this conformation as the "open" state, which in most embodiments is the "on" state. Separation is driven by the thermodynamics of the formation of the target complement sequence-target sequence helix. Formation of the target complement sequence-target sequence helix, whether complete or nicked, overcomes the attraction of the affinity pair under assay conditions. A signal is generated because the separation of the affinity pair alters the interaction of the label moieties and one can thereafter measure a difference in at least one characteristic of at least one label moiety conjugated to the probe. An important feature of the probes for use in this invention is that they do not shift to the open conformation when non-specifically bound.

Probes for use in this invention have a measurable characteristic, which we sometimes refer to as a "signal", that differs depending on whether the probes are open or closed. The measurable characteristic is a function of the interaction of the label moieties and the degree of interaction between those moieties varies as a function of their separation. As stated, unitary probes for use in this invention have a closed conformation and an open conformation. The label moieties are more separated in the open conformation than in the closed conformation, and this difference is sufficient to produce a detectable change in at least one measurable characteristic. In the closed conformation the label moieties are "proximate" to one another, that is, they are sufficiently close to interact so that the measurable characteristic differs in detectable amount, quality, or level, from the open conformation, when they do not so interact. It is desirable, of course, that the difference be as large as possible. In some cases it is desirable that in the "off" state the measurable characteristic be a signal as close as possible to zero.

The measurable characteristic may be a characteristic light signal that results from stimulating at least one member of a fluorescence resonance energy transfer (FRET) pair. It may be a color change that results from the action of an enzyme/suppressor pair or an enzyme/cofactor pair on a substrate to form a detectable product. In all of these cases, we say that the probes have a characteristic signal whose level depends on whether the label moieties are proximate due to the probes being in the closed position or are separated due to the probes being in the open position.

As stated, a detectable signal may be generated by the probe in either the open or closed conformation. The choice of label moieties dictates in which state a signal is generated or that different signals are generated in each state.

Interactive label moieties are a fluorophore/quencher pair, covalently conjugated to the probe, to arm portions that are not complementary to the target. Probes thus generate a positive fluorescent signal of a particular wavelength when bound to the target in the open state and stimulated with an appropriate light source. When referring to these probes we also refer to this conformation as the "on" state.

Also useful with this invention are "universal stems" and kits. A universal stem may be used to construct probes for use with this invention for the detection of one or another target sequence by ligating or otherwise covalently linking to the universal stem an oligonucleotide or oligonucleotides containing a sequence complementary to the desired target sequence.

The invention further comprises assay methods which utilize at least one unitary probe according to this invention. Assays of this invention may be used for targets that are single-stranded or double-stranded. However, for assays that include a step or steps that may separate the affinity pair in a target-independent manner, only unimolecular probes are suitable. Assays according to this invention may be performed in vitro or in vivo. The assays may be performed in situ in living or fixed tissue without destruction of the tissue. Preferred assays of this invention do not require separation or washing to remove unbound probes, although washing may improve performance. Our most preferred assays are homogeneous assays.

Assays according to this invention comprise at least adding at least one unitary probe according to this invention to a sample suspected to contain nucleic acid strands containing a target sequence, under assay conditions appropriate for the probe, and ascertaining whether or not there is a change in the probe's measurable characteristic as compared to that characteristic under the same conditions in the absence of target sequence. The assays may be qualitative or quantitative. In some embodiments, it may be desirable to run a control containing no target and to compare the response of the sample to the response of the control. The level of signal may be measured for quantitative determinations. A change may simply be detected for qualitative assays. When a control is used, the difference in signal change between the sample and the control may be calculated.

Assays according to this invention may include contacting at least one unimolecular probe of the invention with amplification or other nucleic acid synthesis reactions, for example: polymerase chain reactions, PCR, (Erlich et al., 1991); Q-beta replicase-mediated amplification reactions, (Lomeli et al., 1989) ; strand-displacement amplification reactions, SDA, (Walker et al., 1992); self-sustained sequence reactions, 3SR, (Guatelli et al., 1990); and transcription and replication reactions. Bimolecular probes, as stated above, are not suitable for use in any reaction, e.g., PCR, in which the affinity pair would be separated in a target-independent manner; but they may be used in other reactions, e.g., transcription. In any such reaction we prefer unimolecular probes, however. These assays may be qualitative or quantitative. They may detect synthesized target in real-time mode. Of course, either unimolecular or bimolecular probes for use in the invention can be used in assays of completed reactions.

This invention also provides a means for locating and isolating amplified target. For example, utilizing a preferred probe comprising a fluorophore/quencher label pair, PCR products can be identified, quantified and, optionally, isolated after electrophoresis in a gel by stimulating the hybridized probe in the gel, any resulting signal indicating the presence, amount and location of the target. Similarly, this invention provides a means for identifying and, optionally, isolating any desired nucleic acid from a mixture of nucleic acids that is separated by physical means, as by chromatography or electrophoresis.

Kits of reagents and macromolecules for carrying out assays according to this invention may also be used.

In this description we sometimes refer to "probe," "target," "oligonucleotide," "nucleic acid", "strand" and other like terms in the singular. It will be understood by workers in the art that many terms used to describe molecules may be used in the singular and refer to either a single molecule or to a multitude. For example, although a target sequence may be detected by a probe in an assay (and in fact each individual probe interacts with an individual target sequence), assays require many copies of probe and many copies of target. In such instances, terms are to be understood in context. Such terms are not to be limited to meaning either a single molecule or multiple molecules.

### Brief Descriptions of the Drawings

FIG. 1 is a schematic representation of a preferred bimolecular probe according to the invention in the "closed" conformation.
FIG. 2 is a schematic representation of a probe of FIG. 1 in the "open" conformation.
FIG. 3 is a schematic representation of the probe of Example I, our most preferred unimolecular probe.
FIG. 4 is a schematic representation of the use of a universal stem as in Example II.
FIG. 5 is a schematic representation of the unimolecular probe of Example III.
FIG. 6 depicts the thermal denaturation curves of the probes of Examples I and III.
FIG. 7 is a photograph illustrating results of Example V.
FIG. 8 is a graph of the kinetics of hybridization of the probes of Example V.
FIG. 9 is a schematic representation of a unimolecular probe with hairpin sequences in the arms, bound to a target sequence.
FIG. 10 is a schematic representation of a tethered unimolecular probe according to the invention.

### Detailed Description of the Invention

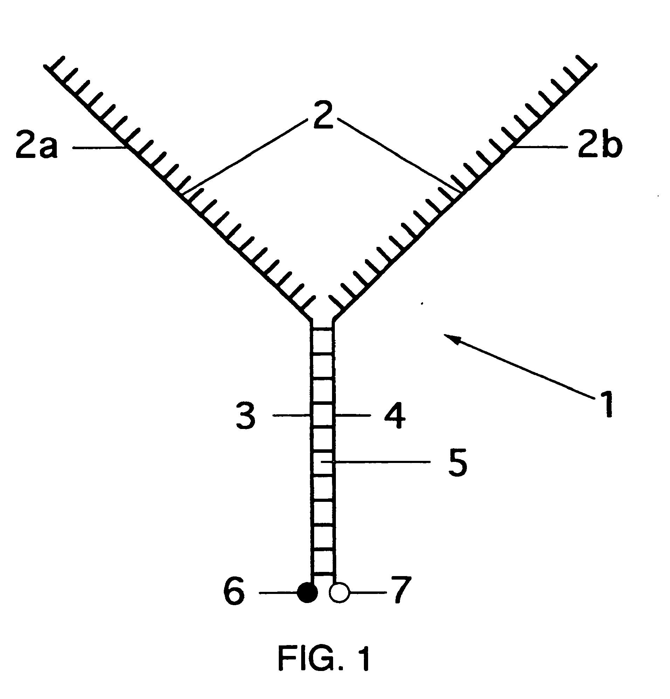

Hybridization probes for use in the invention can be made from DNA, RNA, or some combination of the two. The probes may include modified nucleotides. The links between nucleosides in the probes may include bonds other than phosphodiester bonds. FIG. 1 schematically shows a bimolecular version of unitary probe 1. Probe 1 includes a single-stranded target complement sequence 2 having a 5' terminus and a 3' terminus, which in bimolecular probe 1 includes sequence 2a and sequence 2b, which together are complementary to a preselected target sequence contained within a nucleic acid target strand. Probe 1 can be considered.as a single strand, the unimolecular version, in which a single target complement sequence 2 is severed at about its midpoint. The following description describes probe 1 as so considered, that is, as the unimolecular version, for convenience. The description thus applies to both the bimolecular and unimolecular versions.

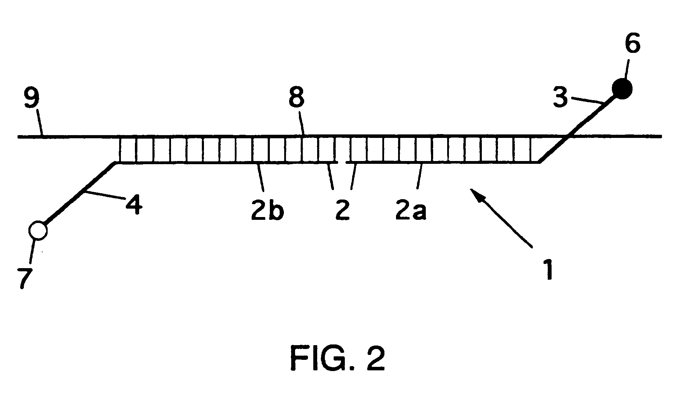

Extending from sequence 2, and linked thereto, are an affinity pair, herein depicted as oligonucleotide arms 3, 4. The affinity pair reversibly interacts sufficiently strongly to maintain the probe in the closed state under detection conditions in the absence of target sequence but sufficiently weakly that the hybridization of the target complement sequence and its target sequence is thermodynamically favored over the interaction of the affinity pair. This balance allows the probe to undergo a conformational change from the closed state to the open state. Additionally, the affinity pair should separate only when probe binds to target and not when probe is non-specifically bound. Referring to FIG. 1, arms 3, 4 are chosen so that under preselected assay conditions, including a detection temperature, they hybridize to each other, forming stem duplex 5, which we sometimes refer to as an arm stem. In the absence of target, association of arms 3, 4 is thermodynamically favored and maintains stem duplex 5, holding the probe 1 in the closed conformation depicted in FIG. 1. In FIG. 2, target complement sequence 2 (comprising sequences 2a and 2b) is hybridized to target sequence 8 of target nucleic acid 9. That hybridization forms a relatively rigid double-helix of appropriate length. For the bimolecular version depicted in FIG. 2, it is a nicked helix. For probes for use in this invention formation of a helix by interaction of the target complement sequence and the target sequence is thermodynamically favored under assay conditions at the detection temperature and drives the separation of arms 3, 4, resulting in dissolution of stem duplex 5 and the maintenance of the open conformation depicted in FIG. 2. Arm regions 3 and 4 do not interact with each other to form the stem duplex when target complement sequence 2 is hybridized to the target sequence 8. Because the interaction of the target complement sequence 2 with the target sequence 8 drives the separation of the arms 3 and 4, we sometimes refer to this mechanism as a "spring." The shift from the closed conformation to the open conformation that occurs when the target complement sequence hybridizes to the target sequence can occur despite the presence of a nick or the presence of one or more nucleotide mismatches. Importantly, non-specific binding of the probe does not overcome the association of the arms in this manner. This feature leads to very low background signal from inappropriately "opened" probes. Arms 3, 4 are chosen so that stem duplex 5 (FIG. 1) is a smaller hybrid than the hybrid of target complement sequence 2 and target sequence 8 (FIG. 2). In the bimolecular version, stem duplex 5 should be smaller than either portion of the nicked helix that includes 2a or 2b. If that limitation is satisfied, we say that each of 2a and 2b contains "approximately half" of target complement sequence 2.

A unitary probe for use in this invention has a measurable characteristic, which we sometimes call a characteristic signal or simply the signal, due to the label pair. Probe 1 includes label moieties 6, 7 conjugated to and forming part of probe 1 at the 5' and 3' termini, respectively, of the stem duplex 5. Label moieties 6, 7 are placed such that their proximity, and therefore their interaction with each other, is altered by the interaction of arms 3, 4. Label moieties 6, 7 could be conjugated.elsewhere to arms 3, 4 or to sequence 2 near its linkage with the stem 5, that is, close to arms 3, 4. Some label moieties will interact to a detectably higher degree when conjugated internally along the arms. This is because they will not be affected by unraveling of the termini.

More than one pair of label moieties may be used. Further, there is no requirement for a one-to-one molecular correspondence between members of a label pair, especially where one member can affect, or be affected by, more than one molecule of the other member. Label moieties suitable for use in probes for use in this invention interact so that at least one moiety can alter at least one physically measurable characteristic of another label moiety in a proximity-dependent manner. The characteristic signal of the label pair is detectably different depending on whether the probe is in the open conformation or the closed conformation.

For example, referring to FIGS. 1 and 2, the preferred label moieties are a FRET pair, most preferably fluorophore 7 and quencher 6. In that embodiment, the characteristic signal is fluorescence of a particular wavelength. When probe 1 is in the closed state (FIG. 1), label moiety 6 quenches fluorescence from moiety 7. When moiety 7 is stimulated by an appropriate frequency of light, a fluorescent signal is generated from the probe at a first level, which may be zero. Probe 1 is "off." When probe 1 is in the open state (FIG. 2), label moiety 6 is sufficiently separated from label moiety 7 that fluorescence resonance energy transfer between them is substantially, if not completely, precluded. Label moiety 6 is therefore unable to quench effectively the fluorescence from label moiety 7. If moiety 7 is stimulated, a fluorescent signal of a second level, higher than the first is generated. Probe 1 is "on." The difference between the two levels of fluorescence is detectable and measurable. Utilizing fluorescent and quencher moieties in this manner, the probe is only "on" in the "open" conformation and indicates that the probe is bound to the target by emanating an easily detectable signal. The conformational state of the probe alters the signal generated from the probe by regulating the interaction between the label moieties.

Lengths of target complement sequences and arm sequences are chosen for the proper thermodynamic functioning of the probe under the conditions of the projected hybridization assay. Persons skilled in hybridization assays will understand that pertinent conditions include probe, target and solute concentrations, detection temperature, the presence of denaturants and volume excluders, and other hybridization-influencing factors. The length of a target complement sequence can range from 10 nucleotides to about 140 nucleotides. For bimolecular embodiments, each portion of the target complement sequence should have a length of at least 10 nucleotides. The lower limit is set by the minimum distance at which there is no detectable difference in the measurable characteristic (or characteristic signal) affected by the interaction between the label moieties used when the probe is closed, from when the probe is opened. Thus, the minimum length of the target complement sequence 2 for a particular probe depends upon the identity of the label pair and its conjugation to the probe. Our most preferred label moieties are the fluorescent moiety 5-[(2-aminoethyl)amino]naphthalene-1-sulfonic acid (EDANS) and quenching moiety 4-(4-dimethylaminophenylazo)benzoic acid (DABCYL). For EDANS and DABCYL, quenching is essentially eliminated by a separation of 60 Angstroms, which is equivalent in length to about 20 nucleotide pairs in a double-helical nucleic acid. Thus, in the preferred embodiment of FIGS. 1 and 2, target complement sequence 2, comprising 2a and 2b, should be at least 20 nucleotides long. Shortening sequence 2 will progressively weaken the signal from hybridized probes and thereby reduce the difference in signal level between open and closed probes.

The maximum length of the probe is less strict and is set by the known flexibility of double-helical nucleic acid molecules (Shore et al., 1981). Because of flexibility in the probe-target hybrid, it is possible for the ends of double-helical nucleic acid molecules to touch each other if the length of the probe-target double-helix exceeds about 140 nucleotide pairs. As will be apparent to those skilled in the art, this distance may vary depending on the nucleic acids in the double-helix (DNA:DNA, DNA:RNA or RNA:RNA) and the type of double-helix formed (A-form, B-form, Z-form, etc.). For example, if the target complement sequence 2 (FIG. 1) is longer than about 140 nucleotides and binds a target to form an A-form DNA:RNA double-helix, undesirable quenching in the open conformation may occur. Occasional quenching, while it may not completely destroy the ability of a probe to generate a detectable signal, tends to degrade probe performance. Undesirable quenching may be reduced by conjugating the label pair to a location other than the termini of the arms.

The maximum length of a target complement sequence is restrained by the functional requirement that a probe assume a thermodynamically favored open conformation when bound to target. Excessively long target complement sequences form probe-target double-helices with sufficient flexibility that both the probe-target helix and the arm stem helix may be present in the same complex. The probe would then remain in the closed state. Therefore, the maximum length of the target complement sequence must be short enough, and the resulting probe-target helix thereby rigid enough, that the probe assumes an open conformation when bound to the target. For the above reasons, the maximum length of the target complement sequence should not exceed in any event about 140 nucleotides, by which we mean within ten percent of that number depending on the factors discussed above.

When designing probes for use in this invention, consideration may be given to the helical nature of double-stranded DNA. When a unimolecular probe is in the open conformation, maximum separation of label moieties is achieved if the moieties are located on opposite sides of the probe-target double-helix. For example, if the label moieties are conjugated to the 5' and 3' termini of the stem duplex distal to the target complement sequence linkage, and a B-form target complement sequence-target sequence double-helix is expected, the choice of a 16-, 26-, 37-, 47-, 58-, 68-, or 79-nucleotide-long target complement sequence will achieve maximum separation by orienting the label moieties in a trans configuration on opposite sides of the double-helix, as label moieties 6, 7 are shown in FIG. 2. In this size range, we prefer target complement sequences which are within 1 to 3 nucleotides of these lengths. We prefer a target complement sequence having a length in the range of 20 to 60 nucleotides. The target complement sequence of our most preferred embodiment constructed to date is 35 nucleotides.

The arm sequences should be of sufficient length that under the conditions of the assay and at the detection temperature, when the probe is not bound to a target, the arms are associated, and the label moieties are kept in close proximity to each other. Depending upon the assay conditions used, 3-25 nucleotide arm lengths can perform this function. An intermediate range of 6-15 nucleotides is often appropriate. The actual length will be chosen with reference to the target complement sequence such that the probe remains in the closed conformation in the absence of target and assumes an open conformation when bound to target. As the target complement sequence increases in size up to 100 nucleotides, the arm length may increase up to 15-25 nucleotides. Above a 100 nucleotide-long target complement sequence, the arm length is not increased further.

The upper limit of the length of the arms is governed by two criteria related to the thermodynamics of probes according to the invention. First, we prefer that the melting temperature of the arm stem, under assay conditions, be higher than the detection temperature of the assay. We prefer stems with melting temperatures at least 5°C higher than the assay temperature and, more preferably at least 10°C higher.

Secondly, the energy released by the formation of the stem should be less than the energy released by the formation of the target complement sequence-target sequence hybrid so that target-mediated opening of the probe is thermodynamically favored. Thus, the melting temperature of the target complement sequence-target sequence hybrid is higher than the melting temperature of the stem. Therefore, arm sequences should be shorter than the target complement sequence. For bimolecular embodiments, as already stated, the arm sequences should be shorter than each portion of the target complement sequence.

Therefore, the melting temperature of the arm stem must be above the assay temperature, so that the probe does not open before the target complement sequence hybridizes to a target, and yet sufficiently below the melting temperature of the hybrid, complete or nicked, of the target complement sequence with the target sequence to assure proper probe functioning and, thereby, generation of a detectable signal. We prefer that the melting temperature of the arm stem be at least 5°C, more preferably at least 10°C, above the assay temperature and at least about 20°C below the melting temperature of the hybrid of the target complement sequence with the target sequence.

One skilled in the art will realize that these parameters will vary with the conditions of the hybridization assay and that those conditions must be considered when designing the nucleic acid sequences of probes for use in this invention. Put another way, the probe must be constructed to function as described above under the conditions of the assay in which it is to be used in order to be a probe for use in this invention. A particular construction may be a probe for use in this invention under one set of assay conditions but not under another set of assay conditions. The length of the arms and their quanosine-cytidine content affect the melting temperature of a stem duplex. For a desired melting temperature, under particular assay conditions, a length and a guanosine-cytidine content of the arms can easily be calculated by those skilled in the art. The melting temperature of the duplex stem of a probe can be empirically determined for given assay conditions using the methods described below in Example IV.

We view these parameters as design considerations which are useful as guidelines. Because the behavior of probes in complex solutions can not always be predicted with certainty, empirical testing is very useful in tailoring probes for use in the invention to perform optimally under particular assay conditions, that is, to maximize the off versus on signal difference and, if desired, to minimize the off signal level.

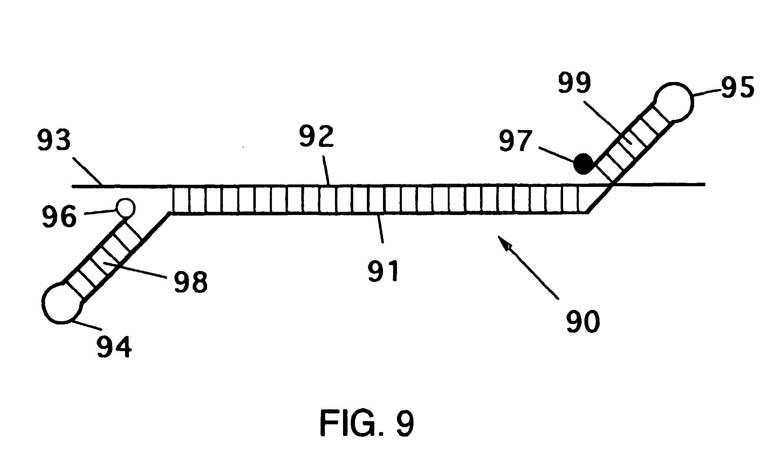

In certain preferred embodiments the sequence of each arm forms an internal secondary structure, such as a hairpin stem, when the probe is open. A unitary probe 90 of this design is illustrated in FIG. 9. Probe 90 is unimolecular, but this design feature applies to bimolecular probes as well. Once target complement sequence 91 has bound to the target sequence 92 of target 93 and formed a probe-target helix, arms 94, 95 are separated, and a signal is generated by separation of label moieties 96, 97. The open conformation shown in FIG. 9 is stabilized because the opened arms 94, 95 fold back on themselves to form hairpins 98, 99. The hairpins are comprised of neighboring complementary sequences that form a stem that is at least three nucleotide pairs in length. This stabilizes the open conformation, because additional energy is released when each separated arm 94, 95 forms an internal hairpin structure. Additionally, arms containing these hairpin structures are effectively shortened and are thus less likely to interact with each other. With this feature it may be possible to use arms, within the 10-25 nucleotide range, that are relatively longer than would be appropriate for arms without this feature to hold the label moieties more tightly together in the closed conformation. Consequently, probes with this feature may exhibit a sharper signal because of a lower level of background in the closed conformation.

Another means of stabilizing the open conformation is to have one or both arm sequences be at least partially complementary to sequences adjacent to the target sequence. Complementary sequences may be placed within an arm at locations proximal or distal to the junction of the arm and target complement sequence. Further, one arm sequence may be completely complementary to sequences adjacent to the target without putting undue restriction on the design of a probe tailored to a specific target. This feature increases the thermodynamic stability of the open conformation. We note that although portions of the arms may be complementary to the target, interaction of the target complement sequence with the target sequence must be sufficient to shift the probe to the open conformation. This design feature applies both to unimolecular embodiments and bimolecular embodiments.

FIGS. 1-5 show our preferred label pair as a luminescent moiety (7, 37, 46, 55) and a quenching moiety (6, 38, 45, 56). Any label pair can be used to generate a signal where one member of the pair can detectably alter at least one physically measurable characteristic of the other when in close proximity, but to a different extent when apart. Alternatively, both members may detectably alter such a characteristic of one member when in close proximity, but differently when apart. Additionally, it is necessary that the label moieties must be conjugatable to the probe.

Luminescent label moieties to be paired with appropriate quenching moieties can be selected from any one of the following known categories: a fluorescent label, a radioluminescent label, a chemiluminescent label, a bioluminescent label and an electrochemiluminescent label. The use of multiple quenching moieties with a single luminescent moiety will increase quenching. In this instance a label pair comprises one fluorescent moiety "paired" to several quenching moieties. Other useful label pairs include a reporter enzyme and appropriate inhibitor.

Although not preferred, a useful label pair may generate a signal in the closed conformation and be inactive in the open conformation. Examples of such pairs are an enzyme and its cofactor and fragments or subunits of enzymes that must be close to each other for the enzyme to be active. In embodiments of this type, the closed conformation is the "on" state.

Our preferred labels are chosen such that fluorescence resonance energy transfer is the mode of interaction between the two labels. In such cases, the measurable physical characteristics of the labels could be a decrease in the lifetime of the excited state of one label, a complete or partial quenching of the fluorescence of one label, an enhancement of the fluorescence of one label or a depolarization of the fluorescence of one label. The labels could be excited with a narrow wavelength band of radiation or a wide wavelength band of radiation. Similarly, the emitted radiation could be monitored in a narrow or a wide range of wavelengths, either with the aid of an instrument or by direct visual observation. Examples of such pairs are fluorescein/sulforhodamine 101, fluorescein/pyrenebutanoate, fluorescein/fluorescein, acridine/fluorescein, acridine/sulforhodamine 101, fluorescein/ethenoadenosine, fluorescein/eosin, fluorescein/erythrosin and anthranilamide-3-nitrotyrosine/fluorescein. Other such label pairs will be apparent to those skilled in the art.

Our most preferred probes, both unimolecular and bimolecular, described more particularly in the Examples, allow detection of hybridization by the naked eye and require only a simple ultraviolet lamp as an excitation device. These probes satisfy the following criteria: only one label moiety is fluorescent, and its fluorescence is visible to the naked eye; the other label moiety quenches this fluorescence extremely efficiently; and no significant quenching occurs at distances greater than about two turns of a nucleic acid double-helix. Of course, multiple copies of one or both label moieties may be used.

Our most preferred fluorescent label is EDANS and our most preferred quenching moiety is DABCYL. The absorption spectrum of DABCYL has a good overlap with the emission spectrum of EDANS, leading to very efficient energy transfer. It has been shown that one can achieve a 40-fold reduction in the fluorescence of EDANS by linking it to DABCYL through an octapeptide spacer, and more than a 200-fold reduction in fluorescence by linking EDANS directly to DABCYL. Also, there is no quenching of the fluorescence of EDANS by DABCYL at distances greater than 60 Angstroms. Finally, DABCYL has no fluorescence of its own (Matayoshi et al., 1990; Wang et al., 1991).

EDANS and DABCYL are conjugated to the probe in the region of the oligonucleotide arms or other affinity pair. In our most preferred probes to date, both unimolecular and bimolecular, EDANS and DABCYL are covalently linked to the free 5' and 3' termini of the arms, distal to the linkage of the arms and the target complement sequence. The positions of EDANS and DABCYL at, respectively, the 5' and the 3' termini can, of course, be reversed. The EDANS and DABCYL moieties could be conjugated anywhere along the terminal portions of the probe, as long as they are proximate to each other in the closed conformation of the probe and sufficiently separated from each other in the open conformation.

Referring to FIG. 1, locating the label moieties along the stem duplex 5, rather than at the free 5' and 3' termini of the arms, may increase the interaction between the label moieties when the probe is in the closed conformation. It is well known that the terminal nucleotides of a double-helix will unravel and rehybridize in a random fashion at a rate dependent on temperature. Therefore, placing the moieties internally along the stem will result in less separation of the moieties due to the unraveling of the termini.

When placing moieties along the stem duplex, consideration may be given to the helical structure of the stem duplex. The moieties may be conjugated to nucleotides staggered along each arm such that when the arms anneal, the moieties will lie on the same side of the stem-duplex helix. This positioning will further maximize interaction of the label moieties in the closed conformation.

As stated earlier, multiple labels, e.g., multiple EDANS and DABCYL moieties, can be used. Multiple labels, in soma cases, permit assays with higher sensitivity. For example, when the affinity pair is oligonucleotide arms, a multiplicity of labels can be achieved by distributing a number of EDANS moieties on one arm and a corresponding number of DABCYL moieties on the other arm, such that each EDANS moiety can be close to a DABCYL moiety when the arm stem helix forms. Multiplicity can also be achieved by covalently linking multiple labels to the arms, in a manner resembling a bunch of grapes. A multiplicity of labels should not be used with label moieties that self-quench. In a preferable application, quenching in the closed conformation may be enhanced by placing a single EDANS moiety on one arm and multiple DABCYL moieties on the other arm such that when the stem helix is formed, at least one DABCYL moiety is adjacent to an EDANS moiety at any given instant.

The conjugation of the label moieties to any location on the probe must be stable under the conditions of the assay. Conjugation may be covalent, which we prefer. Examples of noncovalent conjugation include, without limitation, ionic bonding, intercalation, protein-ligand binding and hydrophobic and hydrophilic interactions. Appropriately stable means of association of label moieties to the probes will be apparent to those skilled in the art. The use of the term "conjugation" herein encompasses all means of association of the label moieties to the probe which are stable under the conditions of use. We consider stably conjugated label moieties to be included within the probe molecule to which they are conjugated.

We sometimes conjugate label moieties to the probes by covalent linkage through spacers, preferably linear alkyl spacers. The nature of the spacer is not critical. For example, EDANS and DABCYL may be linked via six-carbon-long alkyl spacers well known and commonly used in the art. The alkyl spacers give the label moieties enough flexibility to interact with each other for efficient fluorescence resonance energy transfer, and consequently, efficient quenching. The chemical constituents of suitable spacers will be appreciated by persons skilled in the art. The length of a carbon-chain spacer can vary considerably, at least from 1 to 15 carbons. However, in the case of multiple labels conjugated to an arm in a "bunch of grapes" configuration, a multiply bifurcated spacer is desirable.

Hybridization probes and universal stems useful in this invention may comprise nucleic acid molecules that can be assembled by commonly known methods of solid-phase synthesis, by ligation of synthetic sequences or restriction fragments or by a combination of these techniques. The simplest probes can be assembled by synthesis of a single oligonucleotide comprising arm sequences flanking the target complement sequence. Label moieties are then conjugated to the termini of the oligonucleotide. A bimolecular probe may be prepared as two separately synthesized, labeled oligonucleotides. A bimolecular probe can be converted to the corresponding unimolecular probe by linking the target complement sequence portions, as by ligation on a splint. Direct synthesis is particularly appropriate in cases where it can be performed with acceptable yield.

One use of a combination of synthesis and ligation is illustrated by the assembly of a unimolecular DNA probe from two directly synthesized oligodeoxynucleotides. One oligonucleotide contains the target complement sequence, a complete first arm sequence covalently linked to one of the label moieties, and a portion of the second arm sequence. The arm and arm portion of this oligonucleotide hybridize to each other. The second oligonucleotide comprises the remainder of the second arm sequence covalently linked to the other label moiety. The second oligonucleotide is complementary to the unhybridized, or overhang region of the first. The two oligonucleotides are annealed to each other. Finally, the probe is assembled by ligation of the annealed complex.

Alternatively, two oligonucleotides are synthesized, each comprising a substantial portion, "approximately half" as we use that term, of the target complement sequence, an arm sequence located 5' on one oligonucleotide and 3*'* on the other and appropriate label moieties. If a unimolecular probe is desired, these two oligonucleotides are annealed across a splint oligonucleotide and ligated. The probe is then purified from the splint by gel purification or other means known in the art. If a bimolecular probe is desired, the probe is assembled by annealing the two oligonucleotides.

Kits containing a universal stem and instructions to use the universal stem can be used to prepare probes for use in the invention for the detection of a variety of preselected target sequences. The universal stem comprises portions of arm regions, each conjugated to a member of a label pair. For use in making unimolecular probes, a stem may be supplied and used as a unit, with the arms hybridized to each other, such that a linking end of the duplex, comprising reactive groups, is formed. Optionally, the linking end may include a ligatable blunt end or overhang. Alternatively, other biochemical linking agents, or chemical agents, may be present at the 5' and 3' linking termini of the universal stem. Chemical ligation may involve reactive groups, such as hydroxyl, phosphate, sulfhydryl, amino, alkyl phosphate, alkyl amino or hydroxy alkyl. We prefer covalently reactive groups.

Having the universal stem hybridized at the time a probe is prepared is advantageous, because that automatically avoids linking the same arm region at both ends of the probe. Alternatively, however, the universal stem need not be hybridized during probe preparation if mutually exclusive attachment reactions are used for each arm portion. In the latter case, the arm portions may be kept separated for sequential reaction, but we still refer to the arm portions as a universal stem. For use in preparing bimolecular probes, and for use in preparing unimolecular probes by ligation of bimolecular probes, we prefer that the two stem parts be supplied and used separately.

We prefer universal stems comprising oligonucleotide arm portions 5 to 20 nucleotides in length. The nucleotides of the arm portions in a universal stem according to this invention need not be involved in target sequence recognition and will not be except in special cases, as will be understood. The stem need only participate in regulating the conformational state of the unitary probe. Our preferred universal stems include FRET pairs as label moieties, most preferably EDANS and DABCYL, each conjugated to one terminus of the stem duplex, distal to the linking end, as described below. We have prepared the two universal stem oligonucleotides by solid-state synthesis. However, natural sequences of appropriate length and melting temperature may also be adapted for use as stems.

As one skilled in the art will recognize, stems possessing an oligonucleotide comprising an overhang sequence that is complementary to itself can lead to undesirable association of two stems. It is thus preferred, although not required, to avoid the use of oligonucleotides that will form such dimers.

Using a universal stem may include synthesis of an oligonucleotide comprising a target complement sequence flanked by the remaining portions of the arm sequences of the final probe. This oligonucleotide self-hybridizes via the remaining arm portions, creating a linkable terminus. If the universal stem includes an overhang, the oligonucleotide should have an overhang complementary to the overhang on the universal stem. This oligonucleotide is then linked to the universal stem, preferably by enzymatic or chemical ligation, as described above. The universal stem is thereby incorporated into the final probe stem of a unimolecular probe

A universal stem is of particular benefit to a researcher wishing to design a variety of unimolecular or bimolecular probes. Universal stems may be part of a kit comprising one or more stems and instructions for preparing appropriate target complement sequence oligonucleotides, or appropriate restriction fragments, and subsequently linking to a stem. The instructions should describe the portions of the arm sequences, if any, required to flank the target complement sequence and form the appropriate linkable terminus, described above. A kit may include multiple universal stems varying by the melting temperature and/or length of the final probe stem to be formed. A kit could have one common stem oligonucleotide and multiple versions of the other stem oligonucleotide varying in length. Thus, a kit may include stems appropriate for preparation of probes for use in this invention with several lengths of target sequence or with the same target sequence for use under a variety of different preselected assay conditions. Instructions in such a kit would direct the user to the proper universal stem for use with a particular target complement sequence according to the teachings herein. Kits may also optionally include enzymes and reagents, so that the kit user may easily use the universal stems to design and prepare probes for use in the present invention.

Assays according to this invention, which may be qualitative or quantitative, do not require washing to remove unbound probes. An assay according to this invention may thus comprise adding a unitary probe according to this invention to a sample suspected to contain strands containing target sequence and ascertaining whether or not a detectable signal occurs under assay Conditions at the detection temperature. Homogeneous assays are preferred, although probes for use in this invention may be used in heterogeneous hybridization assays. A control without target sequence may be run simultaneously, in which case signal generation of the sample and the control may be compared, either qualitatively or quantitatively by measuring the two and calculating a difference. Assays of this invention include real-time and end-point detection of specific single-stranded or double-stranded products of nucleic acid synthesis reactions, such as transcription, replication, polymerase chain reaction (PCR), self-sustained sequence reaction (3SR), strand-displacement amplification reaction (SDA), and Q-beta replicase-mediated amplification reaction. For assays wherein the unitary probes will be subjected to melting or other denaturation, the probes must be unimolecular.

Quantitative assays can employ quantitating methods known in the art. An end point for a sample may be compared to end points of a target dilution series, for example. Also, readings may be taken over time and compared to readings of a positive or negative control, or both, or compared to curves of one or more members of a target dilution series.

Assays according to the invention include in situ detection, qualitative or quantitative, of nucleic acids in, for example, fixed tissues without destruction of the tissue. Because a large excess of probes can be used without the need for washing and without generation of a large background signal, in situ assays of this invention are particularly useful. In situ hybridization assays, according to this invention include "chromosome painting," for the purposes of mapping chromosomes, and for detecting chromosomal abnormalities (Lichter et al., 1990).

By using multiple probes generating different, non-interfering detectable signals, e.g., fluorescence at different wavelengths or fluorescence and colored product formation, assays of this invention can detect multiple targets in a single assay. Also, multiple probes, each specific for different regions of the same target nucleic acid, yet having the same label pair, can be used in order to enhance the signal. If multiple probes are used for the same target, they should bind to the target such that neighboring probes do not quench each other.

Additionally, special probes for use in this invention can be designed to detect multiple target sequences. If multiple target complement sequences are incorporated into one probe, the probe design must be such that hybridization of any one sequence to its target causes the probe to shift from the closed conformation to the open conformation.

Certain preferred embodiments of assays according to this invention comprise addition of probes for use in this invention to a sample and visualization with the naked eye for detection of a specific target nucleic acid sequence in a complex mixture. By comparison with positive standards or the results obtained with positive standards, visualization can be roughly quantitative. In certain situations, as where information on the size of a nucleic acid is desired, nucleic acid in the sample can first be fractionated by non-denaturing gel electrophoresis, and then the gel itself can be assayed directly. Alternatively, hybridization can be carried out on restriction fragments, either without fractionation or prior to fractionation, as in Example VI.

Thus, with this invention the need for often-used procedures such as Southern and northern blotting (Sambrook et al., 1989), can be eliminated. However, probes for use in the present invention are also very useful in such heterogeneous assays. A major drawback of these assays, the requirement of extensive washing to reduce the background signal from probes which are not hybridized to targets, is ameliorated by the use of probes for use in the present invention.

Probes, both unimolecular and bimolecular, emanate a high level of positive signal only in the target-bound, open conformation and little-to-no signal in the closed conformation. Furthermore, probes do not assume the open conformation unless bound to target, remaining closed when non-specifically bound. As described above, this leads to a background signal that is nonexistent or naturally very low. Therefore, the use of these probes greatly simplifies conventional, heterogeneous assays because either no washing is required or only mild, low stringency washing is used to further reduce any background signal present after hybridization. Additionally, conventional hybridizations may be carried out under generally less stringent conditions.

Probes for use in the invention open very quickly upon interaction with a target sequence. The ability to interact is concentration dependent, as workers skilled in hybridization assays recognize. Assay conditions may be selected in which the probes respond to the presence of target nucleic acids and generate signal very quickly. Because of this, assays according to this invention include real-time monitoring of production of specific nucleic acids. Synthesis processes such as transcription, replication or amplification can be monitored by including probes in the reaction mixture and continuously or intermittently measuring the fluorescence. Probes are used in substantial excess so that the relatively abundant probes find their targets in nascent nucleic acid strands before the targets are sequestered by the binding of complementary strands.

The use of probes in this invention in assays for the identification of products of nucleic acid amplification reactions generally eliminates the need for post-amplification analysis to detect desired products and distinguish desired products from unwanted side reactions or background products. Of course, probes for use in the invention can be added at the end of a synthesis process for end-point detection of products. In assays for monitoring the progress of an amplification reaction, the probes can be present during synthesis. The presence of probes improves the accuracy, precision and dynamic range of the estimates of the target nucleic acid concentration. Reactions in closed tubes may be monitored without ever opening the tubes. Therefore, assays using these probes can limit the number of false positives, because contamination can be limited.

Unimolecular probes for use in the invention are particularly useful in assays for tracking polymerase chain reactions, since the probes according to this invention can open and close with a speed that is faster than the speed of thermal cycling. A probe, that is "off" in the closed position, and includes a target complement sequence having complementarity to an expected amplification product, is included in a polymerase chain reaction mixture. For this embodiment the probe has a melting temperature such that the probe remains closed under the reaction conditions at the annealing temperature of the polymerase chain reaction. The probe may, but need not, remain closed at the extension temperature, if that is higher than the annealing temperature. At the annealing temperature, the target complement sequence hybridizes to its target and the probe generates signal. During the ramp down from the denaturing temperature to the annealing temperature and also at the annealing temperature, melted (i.e., opened) probes which do not find their target close rapidly through an intramolecular event. The signal, e.g. fluorescence, may therefore be read at the annealing temperature. It is also possible to program into the reaction cycle a distinct temperature at which the fluorescence is read. In designing a probe for use in a PCR reaction, one would naturally choose a target complement sequence that is not complementary to one of the PCR primers.

Some probes can be displaced from their target sequence by the annealing of the product's complementary strand, as can PCR primers. This can be overcome by increasing the concentration of the probes or by designing an asymmetry into the synthesis of the product strands in the polymerase chain reaction. In asymmetric amplification, more of the strand containing the target is synthesized relative to the synthesis of the complementary strand.

Similarly, other nucleic acid amplification schemes (see Landegren, 1993, for a review) can be monitored, or assayed, with embodiments of probes of this invention. Appropriate probes can be used, for example, in Q-beta replicase-mediated amplification, self-sustained sequence replication, transcription-based amplification, and strand-displacement amplification reactions, without any modification in the scheme of amplification.

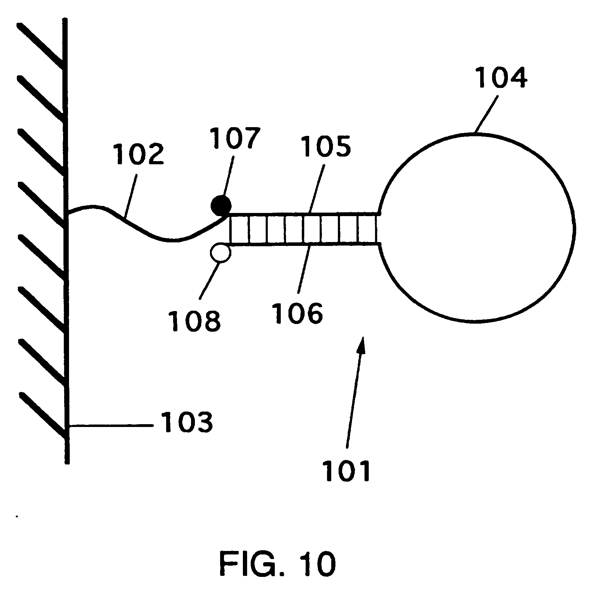

Certain embodiments of assays according to the present invention utilize multiple hybridization probes linked to a solid surface or surfaces. Because probes for use in this invention are used, washing is not required. When probes are linked to a solid surface, we refer to them as "tethered probes." A probe of this type is depicted in FIG. 10, which shows a probe 101 having a target complement sequence 104, complementary arms 105 and 106, and label pair 107, 108 conjugated to arms 105, 106. Probe 101 is tethered to solid surface 103 by a linking moiety 102. Linking moiety 102 may be covalent or noncovalent. We prefer covalent linkage. Any type of surface 103 may be used, including beads, membranes, microtiter wells, and dipsticks. We prefer surfaces that are neutral with respect to the components of the probe, that is, surfaces that do not interact with nucleic acids, do not interact with the label moieties and do not interfere with the probe signal. An example of such a surface is a surface coated with a siliconizing agent.

A useful surface does not significantly interfere with: a) the ability of the affinity pair, preferably arm sequences, of the probe to maintain the closed conformation; b) the hybridization of the target complement sequence to the target; c) the ability of the affinity pair to remain displaced, preferably of arm sequences of the probe to remain unhybridized to each other, when in the open conformation; d) the interaction of proximate label moieties in the closed conformation, preferably the quenching of a luminescent moiety by a quenching moiety; and e) the action of label moieties in the open conformation, preferably the ability of a luminescent moiety to luminescence when stimulated by light of an appropriate frequency. Such surfaces are known in the art.

Probes for use in this invention may be tethered by numerous molecular linking moieties 102 known to those skilled in the art. For example, a suitable linking moiety 102 comprises an alkyl chain or an oligonucleotide chain (such as oligouridine). We prefer oligonucleotide linking moieties because they are easily incorporated into probes. As persons skilled in the art will appreciate, the nucleotide sequence of such an oligonucleotide linking moiety should not be substantially complementary to any other sequence in the probe.

Tethered probes are advantageously useful in assays for the simultaneous determination of a predetermined set of target sequences. For example, a series of luminescent probes can be prepared, each comprising a different target complement sequence. Each probe may be linked to the same support surface, such as a dipstick, at its own predetermined location. After contacting the support and the sample under hybridization conditions, the support may be stimulated with light of an appropriate frequency. Luminescence will occur at those locations where tethered probes have formed hybrids with target molecules from the sample.

Such assays are particularly useful in clinical situations, e.g., in which a patient has an obvious infection, and the physician needs to know the identity of the infectious agent in order to prescribe effective treatment rapidly.

Assay kits for use in this invention include at least one probe and instructions for performing an assay. Kits may also include assay reagents, e.g., salts, buffers, nuclease inhibitors, restriction enzymes and denaturants. Kits may include a target or model target for a positive control test, and a target-less "sample" for a negative control test.

Amplification assay kits may include, in addition to some or all of the above, primers, nucleotides, polymerases and polymerase templates for the assay and for control assays.

Vital stain kits may include, in addition to probe and instructions, permeabilizing agents, liposome precursors, buffers, salts, counterstains and optical filters.

In situ kits may include, in addition to probe and instructions, fixatives, dehydrating agents, proteases, counterstains, detergents, optical filters and coated microscope slides.

Field kits may include, in addition to instructions, tethered probes according to this invention. At least one probe may be tethered to beads, wells or a dipstick. Multiple probes may be included, including a positive control probe that will hybridize to a component of uninfected samples.

Field kits may include, in addition to instructions, untethered probes according to this invention. Such kits may be for infectious agents or genes. Kits for genes may include negative and, sometimes, positive targets.

Probe constructs may be tested to determine whether they are unitary probes for use in the present invention under particular assay conditions. A test may be designed which is appropriate to the affinity pair and label moieties used in the probe construct. The test should be conducted under the conditions of the hybridization assay, using the detector to be used, and generally comprises the following steps: first, one measures the level of signal that is generated in the absence of target, then one measures the level of signal that is generated when there is an excess of probes in the presence of the minimum level of target which one needs to detect. If the level of signal from the first measurement is in accord with the level expected from proximate label moieties, if the level of signal from the second measurement is in accord with the level expected due to opening of the probes, and if the detector can reliably distinguish between the levels of signal from the two measurements, the construct is a probe for use in this invention in that assay.

The probe construct must pass the test described in Example IV to be a probe for use in this invention.

The following Examples illustrate several embodiments of the invention. They are not intended to restrict the invention, which is not limited to these specific embodiments.

### EXAMPLE I: Synthesis of Probe A

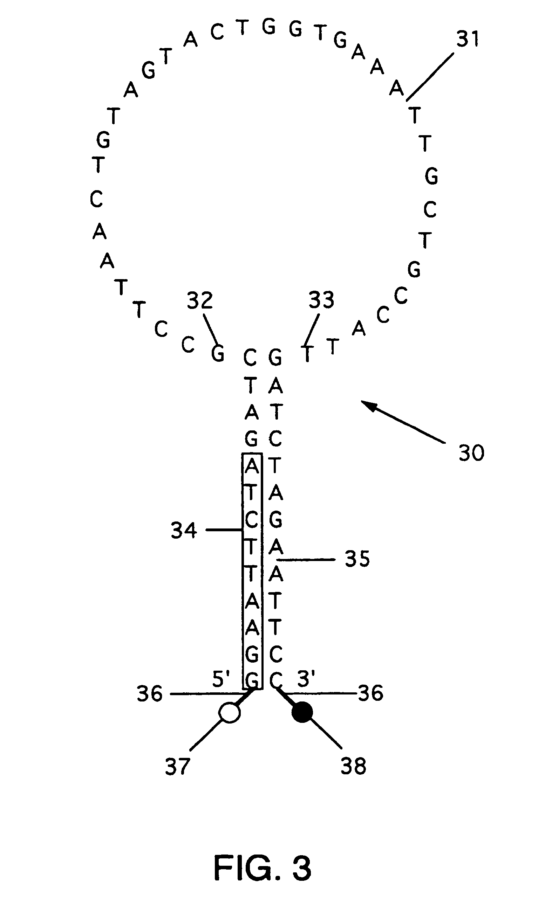

Probe A is depicted in FIG. 3. FIG. 3 shows the nucleotide sequence of a unimolecular probe 30 comprising target complement sequence 31 and complementary arms. The particular probe depicted in FIG. 3 is for detection of the integrase gene of the human immunodeficiency virus HIV-1 (Muesing et al., 1985). Target complement sequence 31, extending, 5' to 3', from nucleotide 32 to nucleotide 33 is complementary to the target sequence 5'-AATGGCAGCAATTTCACCAGTACTACAGTTAAGGC-3'. It will be appreciated that a target complement sequence of the same length, complementary to another target, could be substituted. Probe 30 was assembled using the first two-oligonucleotide ligation method described above. Each of the two oligonucleotides 34, 35 were prepared by solid-state synthesis. During the synthesis of oligodeoxynucleotide 34, identified by a box in FIG. 3, a modified nucleotide was introduced at the 5' terminus. This nucleotide comprises a sulfhydryl group covalently bonded to the 5' phosphate via a hexa-alkyl spacer. Oligonucleotide 34 was then coupled to the EDANS label moiety 37. The method of Connoly and Rider (1985), incorporated herein by reference, was used to couple a sulfhydryl-reactive form of EDANS (1,5-IAEDANS, Molecular Probes, Eugene, Oregon) to the oligonucleotide via a thioether bond. Oligonucleotide 34 was then purified by highpressure liquid chromatography (HPLC). During the synthesis of oligonucleotide 35, the method of Nelson et al., (1989), which is incorporated herein by reference, was used to introduce a primary amino group covalently attached via a hexa-alkyl spacer to the 3' oxygen of the 3'-terminal nucleotide. About 2.5 mg of oligonucleotide 35 was kinased at its 5' end with ³²P using T4 polynucleotide kinase. The ³²P was used to trace the oligonucleotide during synthesis and purification. Kinased oligonucleotide 35 was then dissolved in 300 1 of 0.2 M sodium bicarbonate and reacted with 300 1 60 mg/ml of the amino-reactive form of label moiety 38, DABCYL succinimidyl ester (Molecular Probes, Eugene, Oregon). The amino-reactive DABCYL was added to a continuously stirred reaction mixture in fifteen aliquots of 20 1 each, over a 72-hour period. The reaction mixture was then precipitated with ethyl alcohol. Oligonucleotide 35 was then purified by HPLC. Oligonucleotide 34 and 35, were then annealed to each other and ligated by incubation with T4 DNA ligase, at 16°C. The ligated product was purified by polyacrylamide gel electrophoresis in the presence of 7 M urea. The band containing Probe A showed blue-white fluorescence in the presence of urea (due to the EDANS), was orange in color (due to the DABCYL), and was radioactive (due to the ³²P). Purified Probe A was eluted from the gel and was stored in 10 mM Tris-HCl, pH 8.0 containing 1 mM EDTA (TE buffer).

### EXAMPLE II: Universal Stem and a Method of Using Same

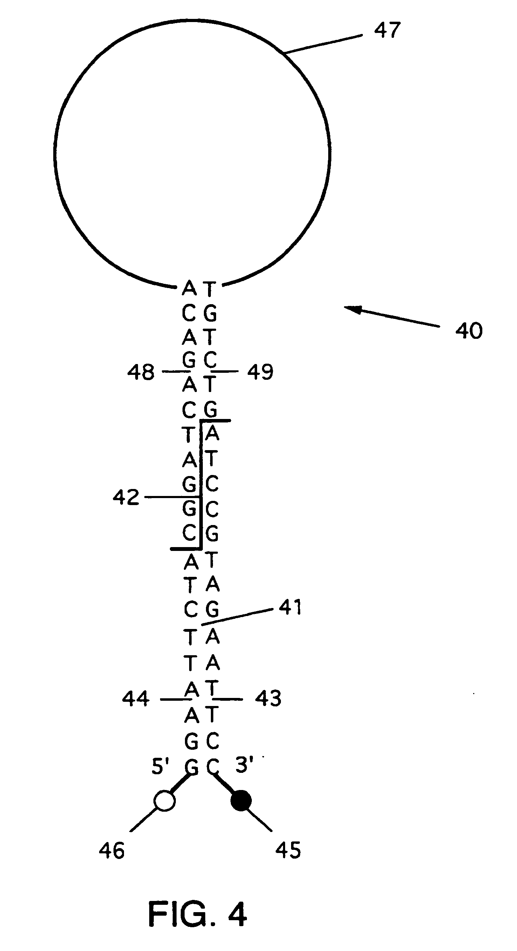

FIG. 4 shows probe 40, also referred to herein as Probe B, designed using a universal stem for use in this invention. Referring to FIG. 4, universal stem 41, delineated by line 42 for clarity, comprises arm regions 43, 44, to which may be ligated a strand containing any target complement sequence, as described below.

To assemble universal stem 41, two oligonucleotides 43, 44 were produced by solid-state synthesis. Oligonucleotide 43 was kinased using T4 polynucleotide kinase. Oligonucleotides 43, 44 are complementary. One, in this case oligonucleotide 43, is 5 nucleotides longer than the other, oligonucleotide 44. Referring to FIG. 4, those five nucleotides are 5'-ATCCG. Oligonucleotide 43 was conjugated to label moiety 45, a DABCYL moiety, at its 3' terminus. Oligonucleotide 44 was conjugated to label moiety 46, an EDANS moiety, at its 5' terminus. These conjugations were performed and the conjugated oligonucleotides were purified as described in Example I. Oligonucleotides 43, 44 were then annealed, thereby placing label moieties 45, 46 in close proximity to each other.

Oligonucleotide 47, prepared by solid-state synthesis, includes a target complement sequence, depicted as a line in FIG. 4, flanked by regions comprising the remaining portions 48, 49 of the arm sequences. Arm portions 48, 49 were annealed to each other to form an overhang complementary to the overhang on the universal stem. The overhang, 5'-CGGAT, comprises the first five nucleotides of arm portion 48. Oligonucleotide 47 was then annealed to universal stem 41 and ligated to it, under the conditions of Example I. Probe B was isolated from the ligation mixture by gel electrophoresis. Although stem sequences 43, 44 and arm portions 48, 49 were annealed separately prior to being mixed, they could have been annealed after mixing. The final arm stem of probe 40 comprises the combination of regions 44 and 48 and regions 43 and 49, respectively.

### EXAMPLE III: Synthesis of Probe C

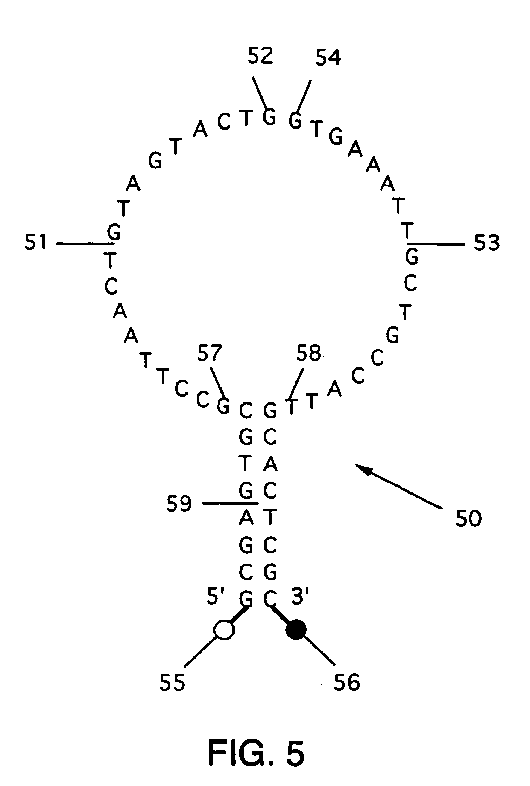

Probe C, a unimolecular probe depicted in FIG. 5, was constructed by the second two-oligonucleotide method described above. Oligonucleotide 51, extending from the 5' end of probe 50 to nucleotide 52, and oligonucleotide 53, extending from nucleotide 54 to the 3' terminus of probe 50 were prepared by solid-state synthesis. Oligonucleotide 51 was conjugated to EDANS 55 at its 5' terminus and oligonucleotide 53 was conjugated to DABCYL 56 at its 3' terminus. The conjugations and purification of the conjugated oligonucleotides were performed as described in Example I.

At this point, the two molecules, oligonucleotides 51 and 53 can be annealed to form a bimolecular probe. A unimolecular probe was formed from these oligonucleotides by annealing them to an oligonucleotide splint having the sequence 5'-AATGGCAGCAATTTCACCAGTACTACAGTTAAGGC-3' and ligated at the junction of nucleotides 52 and 54. The unimolecular probe was then electrophoretically purified as described in Example I. Probe C has the same target complement sequence as Probe A but was designed for use under different conditions. The target complement sequence extends, 5' to 3', from nucleotide 57 to nucleotide 58. The stem duplex 59 of probe C is eight basepairs long.

### EXAMPLE IV: Testing Probe Constructs

This example describes the test to determine whether or not a probe constructed with nucleic acid arm sequences is a probe for use in this invention under particular assay conditions. Data for one such probe construct, Probe A, is provided and evaluated. Probe A was found to be a probe for use in this invention in an assay at 20°C without any salt.

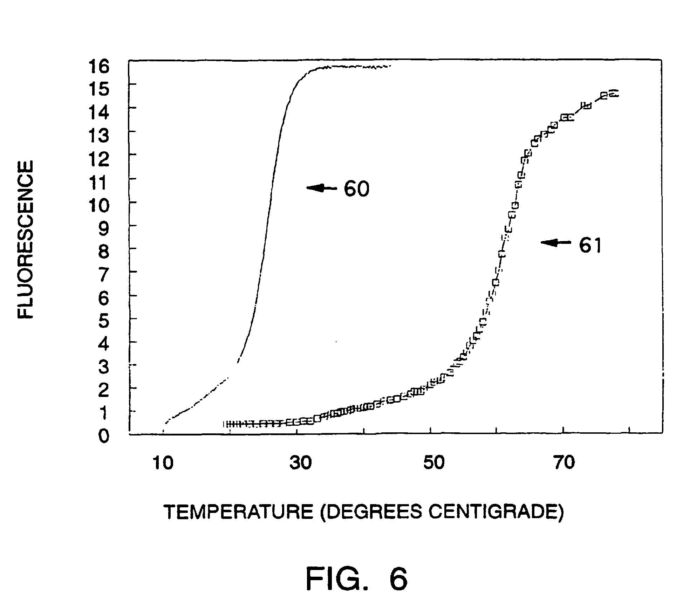

Probes exhibit a characteristic melting temperature, Tm, the temperature at which two hybridized nucleic acid strands separate due to thermal energy. The melting temperature of Probe A was determined by monitoring the level of its fluorescent signal as temperature was increased from 10°C to 80°C under preselected assay conditions: TE buffer with no added salt. The concentration of probes was 150 pmoles in a volume of 2 ml TE buffer. Thermal denaturation or transition curves were recorded with a Perkin-Elmer LS-5B fluorometer. The EDANS moiety of Probe A was excited at 336 nm, and the level of fluorescence at 480 nm was monitored. The results are shown in FIG. 6. Instrumental tracing 60 represents the thermal denaturation curve of Probe A under these assay conditions. The Tm of a probe is indicated by the inflection point of its thermal denaturation curve. The melting temperature, Tm, of Probe A was 27°C.

The following levels of signal, here fluorescence, are noted from the thermal denaturation curve: a first level at Tm-10°C, a second level at Tm+10°C, and a third level at the detection temperature of the preselected assay. A probe construct is for use in this invention under the preselected assay conditions if under those conditions at the detection temperature, addition of excess of model target results in a change in the signal level in the direction toward the level at Tm+10°C by an amount equal to at least ten percent of the difference between the signal levels at Tm-10°C and Tm+10°C. "Model target" is a nucleic acid strand containing a sequence complementary to the target complement sequence of the probe construct and no more than one additional nucleotide immediately adjacent, 5' or 3', thereto. Often, actual target will meet this definition. The concentration of probe constructs used to measure the signal level in the presence of model target is the same concentration used to measure the signal levels in the absence of target, i.e., the concentration used to obtain the thermal denaturation curve.

For Probe A, signal levels were determined from instrumental tracing 60 presented in FIG. 6, as follows: at Tm-10°C, a level of 2 units; at Tm+10°C, a level of 16 units; and at the detection temperature, 20°C, a level of 2.5 units. Addition of excess target at the detection temperature shifted the level from 2.5 units to 14.5 units in 3 hours using a large excess of model target, as described below. Thus, Probe A is a probe for use in this invention under these preselected assay conditions.

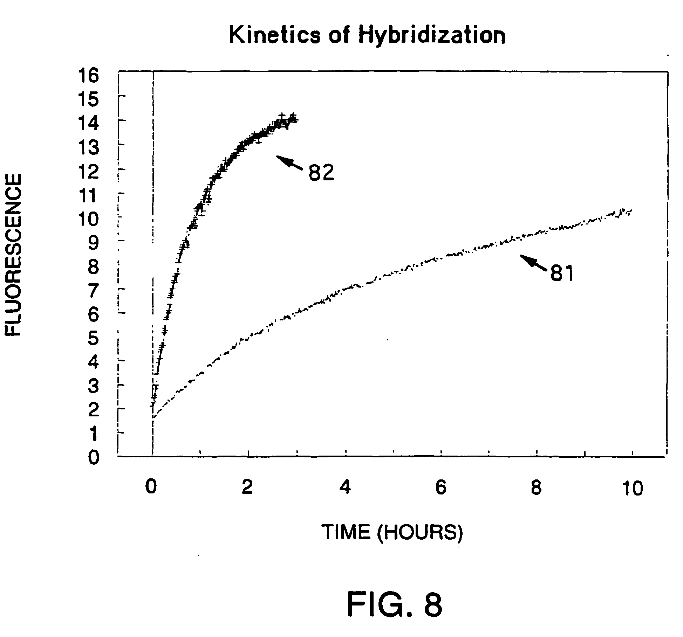

The model target was a DNA strand having the sequence: 5'-CAGACAATGGCAGCAATTTCACCAGTACTACAGTTAAGGCCGCCTGT-3' which was produced by solid-state synthesis and purified by HPLC. The underlined subsequence identifies the target sequence for Probe A. Model target, 25 nmoles, was added to 2 ml TE buffer containing 150 pmoles of Probe A. Fluorescence was monitored over time in a Perkin-Elmer LS-SB fluorometer with a quartz cuvette maintained at the detection temperature. Instrumental tracing 82 in FIG. 8 shows the results. FIG. 8 shows that a high rate of change of fluorescence occurred early, diminished over time and approached a plateau. The test point is the plateau level, although, as in this case, one need not wait that long if a "test passing" level is achieved earlier. As will be recognized, the time to the plateau level depends on the concentration of model target. For example, when we reduced the model target concentration five-fold, instrumental tracing 81 in FIG. 8, the plateau was not reached even after ten hours.

Another construct, Probe C, was tested under different preselected assay conditions, 10mM MgCl₂ in TE buffer at 37°C. Its thermal denaturation curve, instrumental tracing 61 in FIG. 6, gives its Tm as 61°C. From the data in instrumental tracing 61, and from measurement of signal in the presence of excess model target at 37°C, Probe C was found to be a probe for use in this invention under the preselected assay conditions. A bimolecular embodiment of unitary Probe C was tested under the same assay conditions used to test the unimolecular embodiment of unitary Probe C. The bimolecular probe was also found to be useful in the present invention. Bimolecular probes exhibit virtually the same hydridization kinetics as their unimolecular counterparts.

### EXAMPLE V: Demonstration of Probe Function

Additional tests with Probe A were run to demonstrate probe function. Probe A was tested with an excess of DNA target and with an excess of RNA target. The DNA target was the model target described above. The RNA target was an 880-nucleotide RNA corresponding to the integrase gene of HIV-1 (Muesing et al., 1985). The sequence of the DNA model target is contained within the sequence of the RNA target.

The target nucleic acids and Probe A were suspended in TE buffer. Five 0.5 ml plastic snap-capped tubes were prepared containing: 1) 1,000 pmoles of DNA target; 2) 80 pmoles of RNA target; 3) 80 pmoles of RNA target and 15 pmoles of Probe A; 4) 1,000 pmoles of DNA target and 15 pmoles of Probe A; and 5) 15 pmoles of Probe A. The final volume of each test was adjusted to 6 µl with TE buffer.

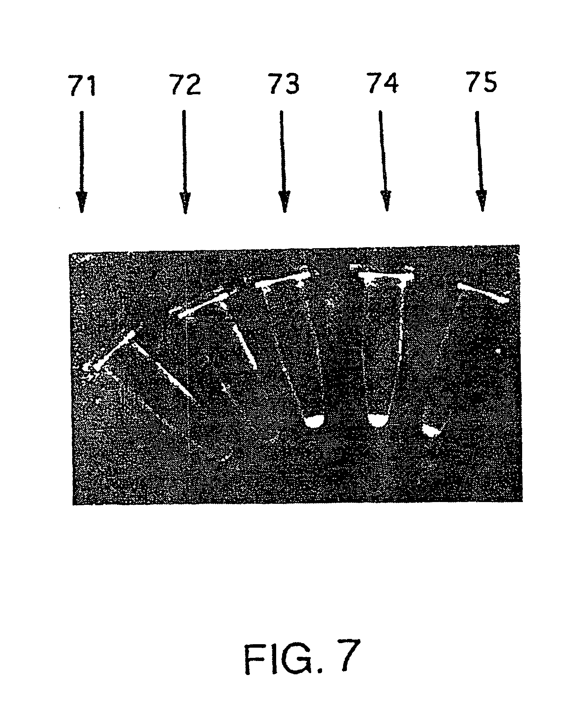

While illuminating the tubes with ultraviolet light from a Transilluminator (Model No. 3-3100, Fotodyne, New Berlin, Wisconsin) the tubes were mixed by gently pipetting twice with a Gilson micropipettor. Intense blue fluorescence, indicating detection of the targets by Probe A, was observed by eye and photographed. Results 71-75, corresponding to tubes 1-5, respectively, are shown in FIG. 7.

The appearance of a fluorescent signal was virtually instantaneous in the tube containing the DNA target and occurred in several minutes in the tube with the RNA target. The delay with the RNA target is believed due to the lower amount of target used, although it could be due to sequestering of the target sequence by the surrounding sequences in the RNA. In a control test, unrelated nucleic acids were mixed with Probe A. No fluorescence was observed in these controls (data not shown).

From the results of these experiments, we infer that: a) in the absence of target, well-designed probes for use in this invention a very low level of background signal; b) probe-target hybridization can produce a signal change detectable by the human eye; c) probes for use in this invention work with either RNA or DNA targets; and d) in the presence of target, probes for use in this invention can turn "on" very rapidly, within a few seconds. Additionally, rapid heating and cooling of Probe A in the absence of target sequence demonstrated that the probe also turned "off" very rapidly, virtually instantaneously.

The sample used in the test reported in FIG. 8, tracing 82, was subsequently heated to 95°C, rapidly cooled in an ice bath, and incubated again at 20°C, the detection temperature. The kinetics of signal generation was followed as before. The tracing during incubation at 20°C was virtually indistinguishable from tracing 82. This demonstrates that with well designed unimolecular probes, probe-target hybrids are reversibly thermally denaturable. Thus, those embodiments are suitable for incorporation into thermal cycling reactions, such as PCR, for real-time detection of amplification products.

### EXAMPLE VI: Assays

Assays that utilize the probes for use in this invention begin simply by addition of the probes to the material of interest under conditions that are conducive to hybridization. The methods of processing the samples and monitoring the fluorescence signal may vary with the nature of the samples. Tissues may be disrupted mechanically or by incubation with chaotropic salts. Most disrupted tissues may be used directly in the assays. Some tissues, however, contain naturally fluorescent materials that may interfere with the detection of signal. In such cases, the nucleic acids may be isolated from the fluorescent materials either before or after hybridization. The fluorescence of opened probes can be monitored by fluorometers.

Preferred unitary probes are useful in field tests for infectious diseases. For example, a test for malaria may begin by addition of guanidine thiocyanate to a sample of blood to lyse the cells, detoxify the cells and denature the constituents. A large excess of probe (relative to the expected maximal target concentration) which is complementary to the ribosomal RNA of the malarial parasite may then be added, and hybridization allowed to proceed. Fluorescence of open probes may be monitored either visually or with help of a fluorometer. Detection of a positive fluorescent signal indicates an infection by the malarial parasite.

Probes can be used to locate particular nucleic acid fragments in a gel or other medium, for example where information on the size of a specific nucleic acid is desired. The nucleic acids in the sample can first be fractionated by gel electrophoresis and then the gel itself bathed in a solution containing the probes. The location in the gel where the target nucleic acid migrates will be detectable by the characteristic signal as a result of hybridization.

Probes preferably unimolecular, having a fluorescer and quencher as the label moieties, can be used as vital stains for the detection of cells that harbor a target nucleic acid. Modified nucleotides are especially useful for such probes. In order to deliver the probes into the cells, a permeabilizing agent, such as toluene, is added to the tissue prior to the addition of the probes. Alternatively, probes can be encapsulated into liposomes and these liposomes fused with the cells. After the delivery of probes, hybridization takes place inside the cells. When the tissue is observed under a fluorescent microscope, the cells that contain the target nucleic acid will appear fluorescent. The subcellular location of a target nucleic acid can also be discerned in these experiments.

Production of nucleic acids in synthesis reactions may be monitored by including appropriately designed probes in the reaction mixture and monitoring the level of signal, e.g., fluorescence, in real-time. The probes should be designed to be complementary to a segment of the nucleic acid that is produced. Examples of such reactions are RNA synthesis by DNA-dependent RNA polymerases and by Q-beta replicase. Unimolecular probes are particularly useful in tracking a polymerase chain reaction, since they open and close with a speed faster than the speed of thermal cycles used in this reaction. An additional temperature in each cycle, which is 5-12°C lower than the melting temperature of the stem of the probe, may be included as the detection temperature. In each cycle, the level of fluorescence will indicate the amount of target DNA strand present. An excess of the probes, as an excess of PCR primers, in the reaction mixture should be used. The PCR may be asymmetric. Real-time monitoring of the correct products, as opposed to end-point detection, improves the precision and the dynamic range of the estimates of the target nucleic acid concentrations by polymerase chain reactions and obviates the need for post-amplification analysis.

The probes may also be used for monitoring other nucleic acid amplification reactions, such as strand displacement amplification reactions and self-sustained sequence replication reactions. Useful probes are designed and used in a manner similar to the probes for polymerase chain reaction products.

### EXAMPLE VII: Tethered Probes

Probes may be used in assays wherein they are tethered to a solid support as discussed above and depicted in FIG. 10.

In a preferred embodiment, multiple probes are prepared. Each probe contains a unique target complement sequence. All may contain the same label pair, a fluorophore and a quencher. Each probe also includes an oligonucleotide chain extending from the end of one arm.

Each probe is tethered via an oligonucleotide chain to a specific location on a dipstick assigned to it. This design leaves the target complement sequences free to participate in homogeneous hybridization. In an assay utilizing this embodiment, the dipstick is contacted with a sample that may contain one or several different target sequences.

The tethered probes then interact with their corresponding target sequences. Those probes so interacting will shift to the open state. The dipstick is illuminated with light of an appropriate frequency. Fluorescence from particular locations on the dipstick indicates the presence of corresponding target sequences. Additional configurations of tethered probe assays will be apparent to those skilled in the art.

### REFERENCES

Cardullo, R.A., Agarwal, S., Flores, C., Zamecnik, P.C. and Wolf, D.E., (1988), Detection of hybridization by nonradiative fluorescence energy transfer, Proc. Natl. Acad. Sci. U.S.A. 85, 8790-8794.
Connoly, B.A. and Rider, P., (1985), Chemical synthesis of oligonucleotide containing a free sulphydryl group and a subsequent attachment of thiol specific probes, Nucleic Acids Res. 13, 4485-4502.
Diamond, S.E., Brewen, J.G., Williams, J.I., Ellwood, M.S., Collins, M. and Fritsch, E.F., (1988), Displacement polynucleotide assay method and polynucleotide complex reagent therefor, U.S. Patent No. 4,766,062.
Erlich, H.A., Gelfand, D. and Sninsky, J.J., (1991), Recent advances in the polymerase chain reaction, Science 252, 1643-1651.
Gillespie, D. and Spiegelman, S., (1956), A quantitative assay for DNA-RNA hybrids with DNA immobilized on a membrane, J. Mol. Biol. 12, 829-852.
Guatelli, J.C., Whitfield, K.M., Kwoh, D.Y., Barringer, K.J., Richman, D.D. and Gingeras, T.R., (1990), Isothermal in vitro amplification of nucleic acids by a multienzyme reaction modeled after retroviral replication, Proc. Natl. Acad. Sci. U.S.A. 87, 1874-1878.
Heller, M.J., Morrison, L.E., Prevatt, W.D. and Akin, C., (1983), Homogeneous nucleic acid hybridization diagnostics by nonradiative energy transfer, European Patent Application 070685.
Landegren, U., (1993), Molecular mechanics of nucleic acid sequence amplification, Trends Genet. 9, 199-204.
Lichter, P., Tang, C.J.C., Call, K., Hermanson, G., Evans, G.A., Housman, D. and Ward, D.C., (1990), High resolution mapping of human chromosome 11 by in situ hybridization with cosmid clones, Science 247, 64-69.
Lomeli, H., Tyagi, S., Pritchard, C.G., Lizardi, P.M. and Kramer, F.R., (1989), Quantitative assays based on the use of replicatable hybridization probes, Clin. Chem. 39, 1826-1831.
Matayoshi, E.D., Wang, G.T., Krafft, G.A. and Erickson, J.E., (1990), Novel fluorogenic substrates for assaying retroviral proteases by resonance energy transfer, Science 247, 954-958.
Morrison, L.E., (1987), Competitive homogeneous assays, European Patent Application 87300195.2
Morrison, L.E., (1989), Lifetime-resolved assay procedures, U.S. Patent No. 4,822,733.
Morrison, L.E., Halder, T.C. and Stols, L.M., (1989), Solution phase detection of polynucleotides using interacting fluorescent labels and competitive hybridization, Analyt. Biochem. 183, 231-244.
Morrison, L.E. and Stols, L.M., (1993), Sensitive fluorescence-based thermodynamic and kinetic measurements of DNA hybridization in solution, Biochemistry 32, 3095-3104.
Muesing, M.A., Smith, D.H., Cabrailla, C.D., Benton, C.V., Lasky, L.A. and Eopon, D.J., (1985), Nucleic acid structure and expression of the human AIDS/adenopathy retrovirus, Nature 313, 450-458.
Nelson, P.S., Fry, R.A. and Liu, E., (1989), Bifunctional oligonucleotide probes synthesized using a novel CPG support are able to detect single base pair mutations, Nucleic Acids Res. 17, 7187-7194.
Sambrook, J., Fritsch, E.F. and Maniatis, T., (1989), Molecular cloning--a laboratory manual, Cold Spring Harbor Laboratory Press.
Shore, D., Langowski, J. and Baldwin, R.L., (1981), DNA flexibility studied by covalent closure of short fragments into circles, Proc. Natl. Acad. Sci. U.S.A. 78, 4833-4827.
Walker, G.T., Fraiser, M.S., Schram, J.L., Little, M.C., Nadeau, J.G. and Malinowski, D.P., (1992), Strand displacement amplification--an isothermal, in vitro DNA amplification technique, Nucleic Acids Res. 20, 1691-1696.
Wang, G.T., Matayoshi, E.D., Huffaker, H.J. and Krafft, G.A., (1991), Design and synthesis of new fluorogenic HIV protease substrates based on resonance energy transfer, Tetrahedron Lett. 31, 6493-6496.

## Claims

1. A process for homogenous detection of at least one amplification product of a nucleic acid amplification reaction comprising
adding to a nucleic acid amplification reaction mixture a dual-conformation oligonucleotide hybridization probe having a target complement sequence hybridizable to said at least one amplification product flanked by a pair of complementary arm sequences labeled, respectively, with a fluorophore and a non-fluorescent quencher capable of absorbing fluorescence from said fluorophore by fluorescence resonance energy transfer
performing said nucleic acid amplification reaction ; and
detecting the increase in fluorescence from said fluorophore under conditions at which the target complement sequence hybridizes to said at least one amplification product as an indication of the presence of said at least one amplification product,
wherein during said step of detecting the probe's complementary arms hybridize to one another to form a stem duplex, permitting the quencher to absorb fluorescence emission from the fluorophore but on contact of the probe with the at least one amplification product, within a few seconds hybridization of the probe's target complement sequence to the at least one amplification product overcomes the stem duplex and separates the fluorophore from the quencher,
and wherein said steps of performing the amplification reaction and detecting the at least one amplification product are performed in a closed reaction vessel.

2. The process according to claim 1 wherein the step of detecting is performed in real time during the amplification reaction.

3. The process according to claim 2 wherein the amplification reaction is a polymerase chain reaction comprising repeated temperature cycles of denaturation, annealing and extension temperatures, and wherein during ramping from denaturation temperature to annealing temperature probes not hybridizing to the at least one amplification product rapidly form a stem hybrid.

4. The process according to any of claims 1-3 wherein said non-fluorescent quencher is DABCYL.

## Patentansprüche

1. Verfahren zur homogenen Detektion von mindestens einem Amplifikationsprodukt einer Nukleinsäureamplifikationsreaktion umfassend:
Zugeben einer Dualkonformation-Oligonukleotidhybridisierungssonde zu einem Nukleinsäureamplifikationsreaktionsgemisch, die eine zum Target komplementäre Sequenz aufweist, bzw. hybridisierbar gegen das mindestens eine Amplifikationsprodukt ist, flankiert von einem Paar an komplementären Seitenarm-Sequenzen, markiert mit einem Fluorophor und einem nicht-fluoreszierenden Quentscher, der in der Lage ist, die Fluoreszenz von dem Fluorophor durch Fluoreszenzresonanzenergietransfer zu absorbieren
Durchführen der Nukleinsäureamplifikationsreaktion: und
Detektieren der Zunahme an Fluoreszenz von dem Fluorophor unter Bedingungen, bei denen die zum Target komplementäre Sequenz an das mindestens eine Amplifikationsprodukt bindet, als ein Anzeichen für das Vorhandensein des mindestens einen Amplifikationsprodukts,
wobei während des Schritts des Detektierens die komplementären Seitenarme der Sonde aneinander hybridisieren um einen doppelsträngigen Stamm zu bilden, was dem Quentscher erlaubt, die Fluoreszenzemission von dem Fluorophor zu absorbieren, jedoch bei Kontakt der Sonde mit dem mindestens einen Amplifikationsprodukt innerhalb weniger Sekunden die Hybridisierung der zum Target komplementären Sequenz der Sonde an das mindestens eine Amplifikationsprodukt den doppelsträngigen Stamm überwindet und den Fluorophor von dem Quentscher trennt,
und wobei die Schritte des Durchführens der Amplifikationsreaktion und des Detektierens des mindestens einen Amplifikationsprodukts in einem geschlossenen Reaktionsgefäß durchgeführt werden.

2. Verfahren nach Anspruch 1, wobei der Schritt des Detektierens während der Amplifikationsreaktion in Echtzeit durchgeführt wird.

3. Verfahren nach Anspruch 2, wobei die Amplifikationsreaktion eine Polymerasekettenreaktion ist, umfassend wiederholte Temperaturzyklen von Denaturierungs-, Anlagerungs- und Verlängerungstemperaturen, und wobei während des Rampings von der Denaturierungstemperatur zur Anlagerungstemperatur die Sonden, die nicht an das mindestens eine Amplifikationsprodukt binden, schnell ein Stammhybrid bilden.

4. Verfahren nach einem der Ansprüche 1-3, wobei der nicht-fluoreszierende Quentscher DABCYL ist.

## Revendications

1. Procédé de détection homogène d'au moins un produit d'amplification d'une réaction d'amplification d'acide nucléique, comprenant les étapes consistant à :
ajouter à un mélange réactionnel d'amplification d'acide nucléique une sonde d'hybridation oligonucléotidique à double conformation possédant une séquence complémentaire cible capable de s'hybrider audit au moins un produit d'amplification flanquée d'une paire de séquences de bras complémentaires marquées respectivement avec un fluorophore et un agent d'extinction *(quencher)* non fluorescent capable d'absorber la fluorescence émise par ledit fluorophore par transfert d'énergie par résonance de fluorescence;
effectuer ladite réaction d'amplification d'acide nucléique ; et
détecter l'augmentation de la fluorescence émise par ledit fluorophore dans des conditions dans lesquelles la séquence complémentaire cible s'hybride audit au moins un produit d'amplification en tant qu'indication de la présence dudit au moins un produit d'amplification,
dans lequel, au cours de ladite étape de détection, les bras complémentaires de la sonde s'hybrident l'un à l'autre pour former une tige duplex, permettant ainsi à l'agent d'extinction d'absorber la fluorescence émise par le fluorophore mais, lors du contact avec la sonde, ledit au moins un produit d'amplification, après quelques secondes d'hybridation de la séquence complémentaire cible de la sonde audit au moins un produit d'amplification, franchit la tige duplex et sépare le fluorophore de l'agent d'extinction,
et dans lequel lesdites étapes de réalisation de la réaction d'amplification et de détection dudit au moins un produit d'amplification se font dans un réacteur fermé.

2. Procédé selon la revendication 1, dans lequel l'étape de détection se fait en temps réel au cours de la réaction d'amplification.

3. Procédé selon la revendication 2, dans lequel la réaction d'amplification est une réaction en chaîne de la polymérase comprenant des cycles répétés de températures de dénaturation, de circularisation et d'extension, et dans lequel, au cours de la montée de la température de dénaturation à la température de circularisation, les sondes qui ne s'hybrident pas audit au moins un produit d'amplification forment rapidement une tige hybride.

4. Procédé selon l'une quelconque des revendications 1 à 3, dans lequel ledit agent d'extinction non fluorescent est le DABCYL.
